# EUROPEAN PATENT APPLICATION

(11) **EP 4 458 369 A1**
(43) Date of publication of application: **06.11.2024**
(21) Application number: 22914994.3
(22) Date of filing: 29.12.2022
(51) Int. Cl.: A61K 38/17, C12N 15/11, A61P 25/28, A61P 25/00

(54) **USE OF PROTON PUMP MODULATOR IN PREPARING REAGENT**

(30) Priority: 29.12.2021 CN 202111635028
(71) Applicant: Shanghai Quietd Biotechnology Co., Ltd., Shanghai 201300 (CN)
(72) Inventor: LI, Kaicheng, Shanghai 201300 (CN); LI, Zhen, Shanghai 201300 (CN); YOU, Pu, Shanghai 201300 (CN)
(74) Representative: Weickmann & Weickmann PartmbB
(86) International application number: PCT/CN2022/143119
(87) International publication number: WO 2023/125744

(57) **Abstract**

A proton pump modulator, and a use of the proton pump modulator in preparing a reagent. The reagent is used for improving learning ability, treating cognitive impairment, and/or treating neurodegenerative diseases. Also involved are an ATP6V1B2 modulator and a use thereof in preparing a reagent used for improving learning ability, treating cognitive impairment, and/or treating neurodegenerative diseases.

## Description

### TECHNCIAL FIELD

The present application relates to the field of biomedicine, specifically to use of a proton pump modulator in preparing a reagent.

### BACKGROUND ART

Alzheimer disease (AD) is usually caused by damage to synapses and neurons, and abnormalities in the structure and function of neural circuits. Due to the lack of clarity on the mechanisms of synaptic and neuronal damage, the progress of effective prevention and treatment of AD is very slow.

ATP6V1B2 protein encoded by ATP6V1B2 gene is an important structural protein for ATP hydrolysis-driven proton pumps. It is widely distributed in human tissues, but is more abundant in brain tissues, kidneys, osteoclasts and other parts. It plays an important role in synaptic transmission and lysosomal acidification. Mutations in this gene may lead to DOORS syndrome (susceptible autosomal dominant congenital deafness-onychodystrophy syndrome). Decreased expression of ATP6V1B2 protein may be associated with the pathogenesis of AD.

### SUMMARY

The present application provides a method for improving learning ability of a subject. According to the present application, it is found that ATP6V1B2 is a potential target to improve the learning ability of the subject, and thus a reagent and/or proton pump modulator that can increase the expression level and/or activity of the ATP6V1B2 can be used as potential drugs to improve the learning ability of the subject.

In one aspect, the present application provides use of a proton pump modulator in preparing a reagent, and the reagent is used for improving learning ability.

In another aspect, the present application provides use of a proton pump modulator in preparing a reagent, and the reagent is used for treating cognitive impairment.

In another aspect, the present application provides use of a proton pump modulator in preparing a reagent, and the reagent is used for treating neurodegenerative diseases.

In some embodiments, the reagent improves the expression level and/or activity of proton pump-associated protein in a subject.

In some embodiments, the proton pump modulator improves the expression level and/or activity of the proton pump-associated protein in the subject.

In some embodiments, the expression level of the proton pump-associated protein includes the expression level of a gene encoding the proton pump-associated protein, the transcription level of the gene encoding the proton pump-associated protein, and/or the expression level of the proton pump-associated protein.

In some embodiments, the improvement includes that the expression level and/or activity of the proton pump-associated protein is improved by at least about 10% as compared with the expression level and/or activity of original proton pump-associated protein in the subject.

In some embodiments, the expression level of the proton pump-associated protein is measured by implementing a test selected from the following groups: qPCR, qRT-PCR, hybridization analysis, RNA Northern blotting, dot blotting, in-situ hybridization, gel electrophoresis, capillary electrophoresis, column chromatography, western blotting, immunohistochemistry, immunostaining and mass spectrometry.

In some embodiments, the expression level of the proton pump-associated protein is measured by a substance selected from the following groups: a primer capable of specifically amplifying the gene encoding the proton pump-associated protein, a nucleic acid molecule specifically bound to the gene encoding the proton pump-associated protein, a nucleic acid molecule specifically bound to the proton pump-associated protein, a small molecule specifically bound to the proton pump-associated protein, a probe specifically bound to the proton pump-associated protein, and polypeptide specifically bound to the proton pump-associated protein.

In some embodiments, the reagent improves the expression level and/or activity of ATP6V 1 B2 in the subject.

In some embodiments, the proton pump modulator improves the expression level and/or activity of ATP6V1B2 in the subject.

In some embodiments, the expression level of the ATP6V1B2 includes the expression level of an ATP6V1B2 gene, the transcription level of the ATP6V1B2 gene and/or the expression level of ATP6V1B2 protein.

In some embodiments, the improvement includes that the expression level and/or activity of ATP6V1B2 is improved by at least about 10% as compared with the expression level and/or activity of original ATP6V1B2 in the subject.

In some embodiments, the expression level of the ATP6V1B2 is measured by implementing a test selected from the following groups: qPCR, qRT-PCR, hybridization analysis, RNA Northern blotting, dot blotting, in-situ hybridization, gel electrophoresis, capillary electrophoresis, column chromatography, western blotting, immunohistochemistry, immunostaining and mass spectrometry.

In some embodiments, the expression level of ATP6V 1B2 is measured by a substance selected from the following groups: a primer capable of specifically amplifying the ATP6V1B2 gene, a nucleic acid molecule specifically bound to the ATP6V1B2 gene, a nucleic acid molecule specifically bound to the ATP6V1B2 protein, a small molecule specifically bound to the ATP6V1B2 protein, a probe specifically bound to the ATP6V1B2 protein, and polypeptide specifically bound to the ATP6V1B2 protein.

In some embodiments, the proton pump modulator includes a proton pump agonist.

In some embodiments, the proton pump modulator includes a vesicle-type proton pump agonist.

In some embodiments, the proton pump modulator includes an agonist of the ATP6V1B2 protein.

In some embodiments, the proton pump modulator can be bound to the ATP6V1B2 protein.

In some embodiments, the proton pump modulator can be bound to at least part of sequence of the 288th to 512th amino acid sequence of mouse ATP6V1B2 protein.

In some embodiments, the proton pump modulator can be bound to at least part of sequence of the 288th to 512th amino acid sequence of human ATP6V1B2 protein.

In some embodiments, the proton pump modulator includes nucleic acid or a variant thereof, polypeptide or a variant thereof, and/or small molecule.

In some embodiments, the proton pump modulator can increase the synaptic neurotransmitter release.

In some embodiments, the increase includes that at least about 10% is increased as compared with the original synaptic neurotransmitter release level in the subject.

In some embodiments, the proton pump modulator can increase the release frequency of excitatory postsynaptic current.

In some embodiments, the increase includes that at least about 10% is increased as compared with the release frequency level of the excitatory postsynaptic current in the subject.

In some embodiments, the learning ability includes cognitive ability, motor ability, memory ability, and/or spatial exploration ability.

In some embodiments, the improvement of the learning ability includes that compared with an evaluation score of original learning ability of the subject, the evaluation score of the improved learning ability of the subject is increased by at least about 50%.

In some embodiments, the evaluation score of the learning ability is measured by implementing a test selected from the following groups: a new object cognitive test and a water maze test.

In some embodiments, the new object cognitive test is used for evaluating the cognitive ability, motor ability and/or spatial exploration ability.

In some embodiments, the water maze test is used for evaluating the memory ability, motor ability and/or spatial exploration ability.

In some embodiments, the subject includes mammals.

In some embodiments, the subject includes human.

In some embodiments, the subject includes a patient with non-cognitive impairment and/or a patient with non-neurodegenerative disease.

In some embodiments, the subject includes a patient with neurodegenerative disease and/or a patient with cognitive impairment.

In some embodiments, the subject includes a patient with Alzheimer disease.

In some embodiments, the subject is in an aged stage.

In some embodiments, the cognitive impairment includes mild cognitive impairment (MCI), middle cognitive impairment and late cognitive impairment.

In some embodiments, the cognitive impairment includes cognitive impairment caused by normal aging, Lews Body Dementia (LBD), frontotemporal dementia and/or vascular dementia.

In some embodiments, the diseases induced by the cognitive impairment includes Alzheimer disease, multi-infarct, Parkinson's disease, AIDS and/or Creutzfeldt-Jakob disease (CJD).

In some embodiments, the neurodegenerative disease includes acute neurodegenerative disease and chronic neurodegenerative disease.

In some embodiments, the neurodegenerative disease includes neurodegenerative disease caused by neuronal death and glial cell homeostasis, neurodegenerative disease caused by aging, neurodegenerative disease caused by influence on the CNS cell function, neurodegenerative disease caused by abnormal communication between cells, and/or neurodegenerative disease caused by damage to cell motor.

In some embodiments, the neurodegenerative disease includes Alzheimer disease, Parkinson's disease, multiple sclerosis (MS), amyotrophic lateral sclerosis (ALS), and/or Huntington's disease (HD).

In some embodiments, the reagent is formulated to be suitable for oral administration and/or injection administration.

In some embodiments, the reagent is formulated to be suitable for intravenous injection.

In another aspect, the present application provides use of ATP6V1B2, a functional active fragment thereof, or a nucleic acid molecule encoding same in improvement of learning ability.

In another aspect, the present application provides use of ATP6V1B2, a functional active fragment thereof, or a nucleic acid molecule encoding same in treatment of cognitive impairment.

In another aspect, the present application provides use of ATP6V1B2, a functional active fragment thereof, or a nucleic acid molecule encoding same in treatment of neurodegenerative diseases.

In some embodiments, the ATP6V1B2 is derived from human.

In some embodiments, the ATP6V1B2 includes the amino acid sequence as set forth in SEQ ID NO. 8 or 16.

In some embodiments, the functional active fragment of the ATP6V1B2 has the capacity of specifically binding to the amino acid sequence as set forth in SEQ ID NO. 5.

In some embodiments, the functional active fragment of the ATP6V1B2 includes a truncation of the ATP6V1B2.

In some embodiments, the functional active fragment of the ATP6V1B2 includes at least part of sequence of the 288th-512th amino acid sequence of human ATP6V1B2 protein.

In some embodiments, the functional active fragment of the ATP6V1B2 includes at least part of sequence of the 288th-512th amino acid sequence of mouse ATP6V1B2 protein.

In some embodiments, the functional active fragment of the ATP6V1B2 includes the amino acid sequence as set forth in any one of SEQ ID NO. 10-11.

In some embodiments, the nucleic acid molecule includes the nucleotide sequence as set forth in any one of SEQ ID NO. 9 or 17.

In some embodiments, the learning ability includes cognitive ability, motor ability, memory ability, and/or spatial exploration ability.

In some embodiments, the improvement of the learning ability includes that compared with an evaluation score of original learning ability of the subject, the evaluation score of the improved learning ability of the subject is increased by at least about 50%.

In some embodiments, the evaluation score of the learning ability is measured by implementing a test selected from the following groups: a new object cognitive test and a water maze test.

In some embodiments, the new object cognitive test is used for evaluating the cognitive ability, motor ability and/or spatial exploration ability.

In some embodiments, the water maze test is used for evaluating the memory ability, motor ability and/or spatial exploration ability.

In some embodiments, the subject includes mammals.

In some embodiments, the subject includes human.

In some embodiments, the subject includes a patient with non-cognitive impairment and/or a patient with non-neurodegenerative disease.

In some embodiments, the subject includes a patient with neurodegenerative disease and/or a patient with cognitive impairment.

In some embodiments, the subject includes a patient with Alzheimer disease.

In some embodiments, the subject is in an aged stage.

In some embodiments, the cognitive impairment includes mild cognitive impairment (MCI), middle cognitive impairment and late cognitive impairment.

In some embodiments, the cognitive impairment includes cognitive impairment caused by normal aging, Lews Body Dementia (LBD), frontotemporal dementia and/or vascular dementia.

In some embodiments, the diseases induced by the cognitive impairment includes Alzheimer disease, multi-infarct, Parkinson's disease, AIDS and/or Creutzfeldt-Jakob disease (CJD).

In some embodiments, the neurodegenerative disease includes acute neurodegenerative disease and chronic neurodegenerative disease.

In some embodiments, the neurodegenerative disease includes neurodegenerative disease caused by neuronal death and glial cell homeostasis, neurodegenerative disease caused by aging, neurodegenerative disease caused by influence on the CNS cell function, neurodegenerative disease caused by abnormal communication between cells, and/or neurodegenerative disease caused by damage to cell motor.

In some embodiments, the neurodegenerative disease includes Alzheimer disease, Parkinson's disease, multiple sclerosis (MS), amyotrophic lateral sclerosis (ALS), and/or Huntington's disease (HD).

In another aspect, the present application provides use of an ATP6V1B2 modulator in preparing a reagent, and the reagent is used for improving learning ability.

In another aspect, the present application provides use of an ATP6V1B2 modulator in preparing a reagent, the reagent is used for treating cognitive impairment.

In another aspect, the present application provides use of an ATP6V1B2 modulator in preparing a reagent, the reagent is used for treating neurodegenerative diseases.

In some embodiments, the reagent improves the expression level and/or activity of ATP6V1B2 in the subject.

In some embodiments, the ATP6V1B2 modulator improves the expression level and/or activity of ATP6V1B2 in the subject.

In some embodiments, the expression level includes the expression level of an ATP6V1B2 gene, the transcription level of the ATP6V1B2 gene and/or the expression level of ATP6V1B2 protein.

In some embodiments, the activity improvement includes the improvement of the expression level and/or activity of proton pump-associated protein.

In some embodiments, the improvement includes that the expression level and/or activity of ATP6V1B2 is improved by at least about 10% as compared with the expression level and/or activity of original ATP6V1B2 in the subject.

In some embodiments, the expression level of the ATP6V1B2 is measured by implementing a test selected from the following groups: qPCR, qRT-PCR, hybridization analysis, RNA Northern blotting, dot blotting, in-situ hybridization, gel electrophoresis, capillary electrophoresis, column chromatography, western blotting, immunohistochemistry, immunostaining and mass spectrometry.

In some embodiments, the expression level of ATP6V1B2 is measured by a substance selected from the following groups: a primer capable of specifically amplifying the ATP6V1B2 gene, nucleic acid molecules specifically bound to the ATP6V1B2 gene, nucleic acid molecules specifically bound to the ATP6V1B2 protein, a small molecule specifically bound to the ATP6V1B2 protein, a probe specifically bound to the ATP6V1B2 protein, and polypeptide specifically bound to the ATP6V1B2 protein.

In some embodiments, the ATP6V1B2 modulator includes an agonist of the ATP6V1B2 protein.

In some embodiments, the ATP6V1B2 modulator can be bound to the ATP6V1B2 protein.

In some embodiments, the ATP6V1B2 modulator can be bound to at least part of sequence of the 288th to 512th amino acid sequence of mouse ATP6V1B2 protein.

In some embodiments, ATP6V1B2 modulator can be bound to at least part of sequence of the 288th to 512th amino acid sequence of human ATP6V1B2 protein.

In some embodiments, ATP6V1B2 modulator includes a proton pump agonist.

In some embodiments, ATP6V1B2 modulator includes a vesicle-type proton pump agonist.

In some embodiments, ATP6V1B2 modulator includes nucleic acid or a variant thereof, polypeptide or a variant thereof, and/or small molecule.

In some embodiments, ATP6V1B2 modulator includes polypeptide or the variant thereof.

In some embodiments, the polypeptide or variant thereof can be specifically bound to the ATP6V1B2 protein.

In some embodiments, the polypeptide or variant thereof can increase the synaptic neurotransmitter release.

In some embodiments, the increase includes that at least about 10% is increased as compared with the original synaptic neurotransmitter release level in the subject.

In some embodiments, the polypeptide or variant thereof can increase the release frequency of excitatory postsynaptic current.

In some embodiments, the increase includes that at least about 10% is increased as compared with the release frequency level of the excitatory postsynaptic current in the subject.

In some embodiments, the polypeptide or variant thereof includes the amino acid sequence as set forth in SEQ ID NO. 5.

In some embodiments, the polypeptide or variant thereof includes the amino acid sequence as set forth in any one of SEQ ID NO. 1 and 3.

In some embodiments, the learning ability includes cognitive ability, motor ability, memory ability, and/or spatial exploration ability.

In some embodiments, the improvement of the learning ability includes that compared with an evaluation score of original learning ability of the subject, the evaluation score of the improved learning ability of the subject is increased by at least about 50%.

In some embodiments, the evaluation score of the learning ability is measured by implementing a test selected from the following groups: a new object cognitive test and a water maze test.

In some embodiments, the new object cognitive test is used for evaluating the cognitive ability, motor ability and/or spatial exploration ability.

In some embodiments, the water maze test is used for evaluating the memory ability, motor ability and/or spatial exploration ability.

In some embodiments, the subject includes mammals.

In some embodiments, the subject includes human.

In some embodiments, the subject includes a patient with non-cognitive impairment and/or a patient with non-neurodegenerative disease.

In some embodiments, the subject includes a patient with neurodegenerative disease and/or a patient with cognitive impairment.

In some embodiments, the subject includes a patient with Alzheimer disease.

In some embodiments, the subject is in an aged stage.

In some embodiments, the cognitive impairment includes mild cognitive impairment (MCI), middle cognitive impairment and late cognitive impairment.

In some embodiments, the cognitive impairment includes cognitive impairment caused by normal aging, Lews Body Dementia (LBD), frontotemporal dementia and/or vascular dementia.

In some embodiments, the diseases induced by the cognitive impairment includes Alzheimer disease, multi-infarct, Parkinson's disease, AIDS and/or Creutzfeldt-Jakob disease (CJD).

In some embodiments, the neurodegenerative disease includes acute neurodegenerative disease and chronic neurodegenerative disease.

In some embodiments, the neurodegenerative disease includes neurodegenerative disease caused by neuronal death and glial cell homeostasis, neurodegenerative disease caused by aging, neurodegenerative disease caused by influence on the CNS cell function, neurodegenerative disease caused by abnormal communication between cells, and/or neurodegenerative disease caused by damage to cell motor.

In some embodiments, the neurodegenerative disease includes Alzheimer disease, Parkinson's disease, multiple sclerosis (MS), amyotrophic lateral sclerosis (ALS), and/or Huntington's disease (HD).

In some embodiments, the reagent is formulated to be suitable for oral administration and/or injection administration.

In some embodiments, the reagent is formulated to be suitable for intravenous injection.

In another aspect, the present application provides a method for screening drugs capable of improving learning ability, treating cognitive impairment, and/or treating neurodegenerative diseases of a subject, including the following step: detecting the influence of the candidate drugs on the expression level and/or activity of proton pump-associated protein in the subject; after the candidate drugs are applied, the expression level and/or activity of the proton pump-associated protein are improved, so that the candidate drugs can improve the learning ability, treat cognitive impairment, and/or treat neurodegenerative diseases of the subject.

In another aspect, the present application provides a method for screening drugs capable of improving learning ability, treating cognitive impairment, and/or treating neurodegenerative diseases of a subject, including the following step: detecting the influence of the candidate drugs on the expression level and/or activity of ATP6V1B2 in the subject, wherein after the candidate drugs are applied, the expression level and/or activity of the ATP6V1B2 are improved, and the candidate drugs can improve the learning ability, treat cognitive impairment, and/or treat neurodegenerative diseases of the subject.

In some embodiments, the expression level of the proton pump-associated protein includes the expression level of a gene encoding the proton pump-associated protein, the transcription level of the gene encoding the proton pump-associated protein, and/or the expression level of the proton pump-associated protein.

In some embodiments, the improvement includes that the expression level and/or activity of the proton pump-associated protein is improved by at least about 10% as compared with the expression level and/or activity of original proton pump-associated protein in the subject.

In some embodiments, the expression level of the proton pump-associated protein is measured by implementing a test selected from the following groups: qPCR, qRT-PCR, hybridization analysis, RNA Northern blotting, dot blotting, in-situ hybridization, gel electrophoresis, capillary electrophoresis, column chromatography, western blotting, immunohistochemistry, immunostaining and mass spectrometry.

In some embodiments, the expression level of the proton pump-associated protein is measured by a substance selected from the following groups: a primer capable of specifically amplifying the gene encoding the proton pump-associated protein, nucleic acid molecules specifically bound to the gene encoding the proton pump-associated protein, nucleic acid molecules specifically bound to the proton pump-associated protein, a small molecule specifically bound to the proton pump-associated protein, a probe specifically bound to the proton pump-associated protein, and polypeptide specifically bound to the proton pump-associated protein.

In some embodiments, the expression level of the ATP6V1B2 includes the expression level of an ATP6V1B2 gene, the transcription level of the ATP6V1B2 gene and/or the expression level of ATP6V1B2 protein.

In some embodiments, the improvement includes that the expression level and/or activity of ATP6V1B2 is improved by at least about 10% as compared with the expression level and/or activity of original ATP6V1B2 in the subject.

In some embodiments, the expression level of the ATP6V1B2 is measured by implementing a test selected from the following groups: qPCR, qRT-PCR, hybridization analysis, RNA Northern blotting, dot blotting, in-situ hybridization, gel electrophoresis, capillary electrophoresis, column chromatography, western blotting, immunohistochemistry, immunostaining and mass spectrometry.

In some embodiments, the expression level of ATP6V1B2 is measured by a substance selected from the following groups: a primer capable of specifically amplifying the ATP6V1B2 gene, nucleic acid molecules specifically bound to the ATP6V1B2 gene, nucleic acid molecules specifically bound to the ATP6V1B2 protein, a small molecule specifically bound to the ATP6V1B2 protein, a probe specifically bound to the ATP6V1B2 protein, and polypeptide specifically bound to the ATP6V1B2 protein.

In some embodiments, the learning ability includes cognitive ability, motor ability, memory ability, and/or spatial exploration ability.

In some embodiments, the administration includes oral and/or injection administration.

In some embodiments, the administration includes intravenous injection.

In another aspect, the present application provides a polypeptide or variant thereof capable of modulating expression level and/or activity of ATP6V1B2.

In some embodiments, the polypeptide or variant thereof can increase the synaptic neurotransmitter release.

In some embodiments, the increase includes that at least about 10% is increased as compared with the original synaptic neurotransmitter release level in the subject.

In some embodiments, the polypeptide or variant thereof can increase the release frequency of excitatory postsynaptic current.

In some embodiments, the increase includes that at least about 10% is increased as compared with the release frequency level of the excitatory postsynaptic current in the subject.

In some embodiments, the polypeptide or variant thereof can be bound to the ATP6V1B2 protein.

In some embodiments, the polypeptide or variant thereof can improve the expression level and/or activity of the ATP6V1B2 in the subject.

In some embodiments, the expression level of the ATP6V1B2 includes the expression level of an ATP6V1B2 gene, the transcription level of the ATP6V1B2 gene and/or the expression level of ATP6V1B2 protein.

In some embodiments, the improvement includes that the expression level and/or activity of ATP6V1B2 is improved by at least about 10% as compared with the expression level and/or activity of original ATP6V1B2 in the subject.

In some embodiments, the polypeptide or variant thereof can be bound to at least part of sequence of the 288th to 512th amino acid sequence of mouse ATP6V1B2 protein.

In some embodiments, the polypeptide or variant thereof can be bound to at least part of sequence of the 288th to 512th amino acid sequence of human ATP6V1B2 protein.

In some embodiments, the polypeptide or variant thereof includes the amino acid sequence as set forth in SEQ ID NO. 5.

In some embodiments, the polypeptide or variant thereof includes the amino acid sequence as set forth in any one of SEQ ID NO. 1 and 3.

In some embodiments, the polypeptide or variant thereof includes a multimer.

In some embodiments, the polymer includes a homodimer.

In some embodiments, cysteine in the amino acid sequence of the polypeptide or variant thereof does not have a sulfhydryl blocking modification.

In some embodiments, serine in the amino acid sequence of the polypeptide or variant thereof does not have a phosphorylation modification.

In another aspect, the present application provides a pharmaceutical combination, comprising the reagent according to the present application, and/or, comprising the polypeptide or variant thereof according to the present application.

In another aspect, the present application provides a pharmaceutical composition, including the reagent according to the present application, and/or, including the polypeptide or variant thereof according to the present application, and a pharmaceutically acceptable carrier.

In another aspect, the present application provides use of the polypeptide or variant thereof according to the present application, the reagent according to the present application, the pharmaceutical combination according to the present application and/or the pharmaceutical composition according to the present application in preparing a reagent, and the reagent is used for improving learning ability, treating cognitive impairment, and/or treating neurodegenerative diseases.

In some embodiments, the learning ability includes cognitive ability, motor ability, memory ability, and/or spatial exploration ability.

In some embodiments, the cognitive impairment includes mild cognitive impairment (MCI), middle cognitive impairment and late cognitive impairment.

In some embodiments, the cognitive impairment includes cognitive impairment caused by normal aging, Lews Body Dementia (LBD), frontotemporal dementia and/or vascular dementia.

In some embodiments, the diseases induced by the cognitive impairment includes Alzheimer disease, multi-infarct, Parkinson's disease, AIDS and/or Creutzfeldt-Jakob disease (CJD).

In some embodiments, the neurodegenerative disease includes acute neurodegenerative disease and chronic neurodegenerative disease.

In some embodiments, the neurodegenerative disease includes neurodegenerative disease caused by neuronal death and glial cell homeostasis, neurodegenerative disease caused by aging, neurodegenerative disease caused by influence on the CNS cell function, neurodegenerative disease caused by abnormal communication between cells, and/or neurodegenerative disease caused by damage to cell motor.

In some embodiments, the neurodegenerative disease includes Alzheimer disease, Parkinson's disease, multiple sclerosis (MS), amyotrophic lateral sclerosis (ALS), and/or Huntington's disease (HD).

In some embodiments, the polypeptide, the pharmaceutical combination, and/or the pharmaceutical composition are formulated to be suitable for oral administration and/or injection administration.

In some embodiments, the polypeptide, the pharmaceutical combination, and/or the pharmaceutical composition are formulated to be suitable for intravenous injection.

In another aspect, the present application provides a non-human animal or part thereof, comprising and/or expressing ATP6V1B2 , a functional active fragment thereof, and/or a nucleic acid molecule encoding same.

In some embodiments, the ATP6V1B2 is derived from human.

In some embodiments, the ATP6V1B2 includes the amino acid sequence as set forth in SEQ ID NO. 8 or 16.

In some embodiments, the functional active fragment of the ATP6V1B2 has the capacity of specifically binding to the amino acid sequence as set forth in SEQ ID NO. 5.

In some embodiments, the functional active fragment of the ATP6V1B2 includes a truncation of the ATP6V1B2.

In some embodiments, the functional active fragment of the ATP6V1B2 includes at least part of sequence of the 288th-512th amino acid sequence of human ATP6V1B2 protein.

In some embodiments, the functional active fragment of the ATP6V1B2 includes at least part of sequence of the 288th-512th amino acid sequence of mouse ATP6V1B2 protein.

In some embodiments, the functional active fragment of the ATP6V1B2 includes the amino acid sequence as set forth in any one of SEQ ID NO. 10-11.

In some embodiments, the nucleic acid molecules comprises the nucleotide sequence as set forth in SEQ ID NO. 9 or 17.

In some embodiments, the non-human animal is a gene-edited non-human animal, or derived from the gene-edited non-human animal.

In some embodiments, the non-human animal is a non-human animal subjected to gene knock-in, or a non-human animal derived from gene knock-in.

In some embodiments, the expression of the ATP6V1B2 and/or the functional active fragments thereof is modulated by corresponding endogenous modulatory elements in the genome of the non-human animal.

In some embodiments, the non-human animal is a non-human mammal.

In some embodiments, the non-human animal is selected from: mice, rats, rabbits, apes, monkeys, pigs, cows, sheep, horses, chickens, dogs, alpacas and cats.

In some embodiments, the non-human animal is a rodent or a primate.

In some embodiments, the part includes organs, tissues and/or cells of the non-human animal.

In some embodiments, the subject includes body fluid.

In some embodiments, the body fluid is selected from the following groups: blood, plasma, serum, urine, sweat, tears, saliva, semen and cerebrospinal fluid.

In some embodiments, the plasma includes exosomes.

In some embodiments, the part includes brain or part of the brain.

In some embodiments, the part of the brain includes the brain part selected from the following groups: olfactory bulb, amygdala, basal ganglia, hippocampus, thalamus, hypothalamus, cerebral cortex, medulla oblongata, and cerebellum.

In some embodiments, the part includes olfactory mucosa.

In some embodiments, the part includes part of the central nervous system, part of the peripheral nervous system, skin tissue, muscle tissue, and/or internal organs.

In some embodiments, the central nervous system includes spinal cord.

In some embodiments, the part includes cells selected from the following groups: primary neurons, microglia, astrocytes, oligodendrocytes, macrophages, perivascular epithelioid cells, B cells, T cells, adult stem cells, NK cells, totipotent stem cells, unipotent stem cells, embryonic stem cells, induced pluripotent stem cells, and gametes.

In some embodiments, the gametes include spermatozoa and/or oocytes.

In some embodiments, the part cannot develop into a complete non-human animal.

In some embodiments, offspring is obtained by crossing the non-human animal with another non-human animal, and the other non-human animal may or may not express the ATP6V1B2 and/or the functional active fragment thereof.

In some embodiments, the offspring or part thereof contains and/or expresses the ATP6V1B2 and/or the functional active fragment thereof.

In another aspect, the present application provides an isolated cell, including and/or expressing ATP6V1B2, a functional active fragment thereof, and/or nucleic acid molecules encoding same.

In some embodiments, the ATP6V1B2 is derived from human.

In some embodiments, the ATP6V1B2 and/or the functional active fragment thereof includes the amino acid sequence as set forth in any one of SEQ ID NO: 8, 10-11, 16.

In some embodiments, the gene encoding the ATP6V1B2 and/or the functional active fragment thereof is homozygous or heterozygous.

In some embodiments, the nucleic acid molecules encoding the ATP6V1B2 and/or the functional active fragment thereof includes the nucleic acid sequence as set forth in SEQ ID NO: 9 or 17.

In some embodiments, the expression of the ATP6V1B2 and/or functional active fragment thereof is modulated by corresponding endogenous modulatory elements in the genome of the cells.

In some embodiments, the cells are non-human animal cells.

In some embodiments, the non-human animal is a non-human mammal.

In some embodiments, the non-human animal is selected from: mice, rats, rabbits, apes, monkeys, pigs, cows, sheep, horses, chickens, dogs, alpacas and cats.

In some embodiments, the non-human animal is a rodent or a primate.

In some embodiments, the cell includes cells selected from the following groups: neuroblastoma cells, glioma cells, primary neurons, microglia, astrocytes, oligodendrocytes, macrophages, perivascular epithelioid cells, B cells, T cells, adult stem cells, NK cells, totipotent stem cells, unipotent stem cells, embryonic stem cells, induced pluripotent stem cells, and gametes.

In some embodiments, the gametes include spermatozoa and/or oocytes.

In some embodiments, the cell cannot develop into a complete non-human animal.

In another aspect, the present application provides a tissue, including and/or expressing ATP6V1B2 , a functional active fragment thereof, and/or a nucleic acid molecule encoding same.

In some embodiments, the ATP6V1B2 is derived from human.

In some embodiments, the ATP6V1B2 and/or the functional active fragment thereof includes the amino acid sequence as set forth in any one of SEQ ID NO: 8, 10-11, 16.

In some embodiments, the gene encoding the ATP6V1B2 and/or the functional active fragment thereof is homozygous or heterozygous.

In some embodiments, the nucleic acid molecules encoding the ATP6V1B2 and/or the functional active fragment thereof include the nucleic acid sequence as set forth in SEQ ID NO: 9 or 17.

In some embodiments, the tissue is a gene-edited tissue, or derived from gene editing.

In some embodiments, the tissue is a gene-knocked-in tissue, or is derived from a gene-knocked-in tissue.

In some embodiments, the expression of the ATP6V1B2 and/or the functional active fragment thereof is modulated by corresponding endogenous modulatory elements in the genome of the tissue.

In some embodiments, the tissue is a non-human animal tissue.

In some embodiments, the non-human animal is a non-human mammal.

In some embodiments, the non-human animal is selected from: mice, rats, rabbits, apes, monkeys, pigs, cows, sheep, horses, chickens, dogs, alpacas and cats.

In some embodiments, the non-human animal is a rodent or a primate.

In some embodiments, the tissue includes body fluid.

In some embodiments, the body fluid is selected from the following groups: blood, plasma, serum, urine, sweat, tears, saliva, semen and cerebrospinal fluid.

In some embodiments, the plasma includes exosomes.

In some embodiments, the tissue includes brain or part of the brain.

In some embodiments, the part of the brain includes the brain part selected from the following groups: olfactory bulb, amygdala, basal ganglia, hippocampus, thalamus, hypothalamus, cerebral cortex, medulla oblongata, and cerebellum.

In some embodiments, the tissue includes olfactory mucosa.

In some embodiments, the tissue includes part of the central nervous system, part of the peripheral nervous system, skin tissue, muscle tissue, and/or internal organs.

In some embodiments, the central nervous system includes spinal cord.

In some embodiments, the tissue cannot develop into a complete non-human animal.

In another aspect, the present application provides a cell line or cell culture, derived from the non-human animal or part thereof according to the present application, derived from the offspring according to the present application, derived from the cell according to the present application, and/or derived from the tissue according to the present application.

In some embodiments, the cell culture is a primary cell culture.

In some embodiments, the cell culture includes organoids.

In another aspect, the present application provides a composition, including the non-human animal or part thereof according to the present application, the offspring according to the present application, the cell according to the present application, the tissue according to the present application, and the cell line or cell culture according to the present application.

In another aspect, the present application provides a kit, including the non-human animal or part thereof according to the present application, the offspring according to the present application, the cell according to the present application, the tissue according to the present application, and/or the cell line or cell culture according to the present application, and one or more additional components selected from the following groups: analytical buffers, controls, substrates, standards, assay materials, laboratory supplies, devices, instruments, cells, organs, tissues, and user manuals or instructions.

In another aspect, the present application provides a method for screening drugs capable of improving learning ability, treating cognitive impairment, and/or treating neurodegenerative diseases of a subject, including: applying candidate drugs to the non-human animal or part thereof according to the present application, the offspring according to the present application, the cell according to the present application, the tissue according to the present application, and/or the cell line or cell culture according to the present application; and detecting the influence of the candidate drugs on the expression level and/or activity of ATP6V1B2 in the subject.

In another aspect, the present application provides a method for screening drugs capable of improving learning ability, treating cognitive impairment, and/or treating neurodegenerative diseases of a subject, comprising: (i) applying candidate drugs to the non-human animal or part thereof according to the present application, the offspring according to the present application, the cell according to the present application, the tissue according to the present application, and/or the cell line or cell culture according to the present application; (ii) detecting the influence of the candidate drugs on the expression level and/or activity of ATP6V1B2 in the subject; and (iii) in a case that the expression level and/or activity of the ATP6V1B2 is improved after applying the candidate drugs, determining that the candidate drugs can improve the learning ability, treating cognitive impairment, and/or treating neurodegenerative diseases of the subject.

In some embodiments, the expression level of the ATP6V1B2 includes the expression level of an ATP6V1B2 gene, the transcription level of the ATP6V1B2 gene and/or the expression level of ATP6V1B2 protein.

In some embodiments, the improvement includes that the expression level and/or activity of ATP6V1B2 is improved by at least about 10% as compared with the expression level and/or activity of original ATP6V1B2 in the subject.

In some embodiments, the learning ability includes cognitive ability, motor ability, memory ability, and/or spatial exploration ability.

In some embodiments, the cognitive impairment includes mild cognitive impairment (MCI), middle cognitive impairment and late cognitive impairment.

In some embodiments, the cognitive impairment includes cognitive impairment caused by normal aging, Lews Body Dementia (LBD), frontotemporal dementia and/or vascular dementia.

In some embodiments, the diseases induced by the cognitive impairment includes Alzheimer disease, multi-infarct, Parkinson's disease, AIDS and/or Creutzfeldt-Jakob disease (CJD).

In some embodiments, the neurodegenerative disease includes acute neurodegenerative disease and chronic neurodegenerative disease.

In some embodiments, the neurodegenerative disease includes neurodegenerative disease caused by neuronal death and glial cell homeostasis, neurodegenerative disease caused by aging, neurodegenerative disease caused by influence on the CNS cell function, neurodegenerative disease caused by abnormal communication between cells, and/or neurodegenerative disease caused by damage to cell motor.

In some embodiments, the neurodegenerative disease includes Alzheimer disease, Parkinson's disease, multiple sclerosis (MS), amyotrophic lateral sclerosis (ALS), and/or Huntington's disease (HD).

In another aspect, the present application provides use of the non-human animal or part thereof according to the present application, the offspring according to the present application, the cell according to the present application, the tissue according to the present application, and/or the cell line or cell culture according to the present application in preparing an identification and/or screening system; and the system is used for screening drugs and/or biomarkers capable of improving learning ability, treating cognitive impairment, and/or treating neurodegenerative diseases of a subject.

In another aspect, the present application provides the non-human animal or part thereof according to the present application, the offspring according to the present application, the cell according to the present application, the tissue according to the present application, and/or the cell line or cell culture according to the present application, which are used for screening drugs and/or biomarkers capable of improving learning ability, treating cognitive impairment, and/or treating neurodegenerative diseases of a subject.

In another aspect, the present application provides a method for preparing the non-human animal or part thereof according to the present application, the cell according to the present application, the tissue according to the present application, including: enabling the non-human animal or part thereof, cell or tissue to include and/or express ATP6V1 B2 and/or a functional active fragment thereof.

In another, the present application provides a method for preparing a disease model, including: enabling the non-human animal or part thereof, cell or tissue to include and/or express ATP6V1B2 and/or a functional active fragment thereof.

In some embodiments, the ATP6V1B2 and/or the functional active fragment thereof includes the amino acid sequence as set forth in any one of SEQ ID NO: 8, 10-11, 16.

In some embodiments, the method includes: transfecting the nucleic acid sequence encoding the ATP6V1B2 and/or functional active fragment thereof.

In some embodiments, the nucleic acid sequence encoding the ATP6V1B2 and/or functional active fragment thereof includes the nucleic acid sequence as set forth in SEQ ID NO: 9 or 17.

In some embodiments, the transfecting includes: transfecting adeno-associated virus comprising the nucleic acid sequence.

Those skilled in the art can easily discern other aspects and advantages of the present application from the detailed description below. Only exemplary embodiments of the present application are shown and described in the following detailed description. As those skilled in the art will realize, the contents of the present application enable those skilled in the art to make changes to the specific embodiments disclosed without departing from the spirit and scope of the invention covered by the present application. Accordingly, the drawings and descriptions in the specification of the present application are illustrative only and not restrictive.

### BRIEF DESCRIPTION OF DRAWINGS

The specific features of the invention to which the present application relates are set forth in the appended claims. The features and advantages of the invention to which the present application relates can be better understood by reference to the exemplary embodiments described in detail below and the drawings. A brief description of the drawings is as follows:
FIGS. 1a-1i show effects of polypeptide on excitatory synaptic transmission according to the present application.
FIG. 2 shows improvement of cognitive behavior of AD mice in a new object recognition test by polypeptide through intragastric administration according to the present application.
FIG. 3 shows improvement of learning ability of AD mice in a water maze test by polypeptide through intragastric administration according to the present application.
FIG. 4 shows improvement of cognitive behavior of AD mice in a new object recognition test by polypeptide through intravenous administration according to the present application.
FIG. 5 shows improvement of learning ability of AD mice in a water maze test by polypeptide through intravenous administration according to the present application.
FIGS. 6a-6b show results of co-immunoprecipitation of polypeptide and ATP6V1B2 protein according to the present application.
FIGS. 7a-7e show improvement of learning and memory functions of hippocampus of old animals by overexpression of ATP6V1B2 protein.
FIG. 8 shows effect of a dimer of polypeptide on excitatory synaptic transmission according to the present application.
FIG. 9 shows influence of cysteine-modified polypeptide on excitatory synaptic transmission according to the present application.
FIG. 10 shows influence of serine-modified polypeptide on excitatory synaptic transmission according to the present application.
FIG. 11 shows results of co-immunoprecipitation of polypeptide and ATP6V1B2 protein or truncated fragments thereof according to the present application.

### DETAILED DESCRIPTION OF THE INVENTION

The embodiments of the invention of the present application will be described below with specific examples. Those skilled in the art can easily understand other advantages and effects of the invention of the present application from the disclosure of the specification.

### Definition of Terms

The term "ATP6V1B2" generally refers to ATPase H⁺ transporting V1 subunit B2 protein (or referred to as ATP6B2, DOOD, HO57, VATB, VPP3, Vma2 or ZLS2), and gene encoding this protein. The ATP6V1B2 may be a multimeric enzyme that mediates intracellular organelle acidification in eukaryotic cells. The ATP6V1B2 may participate in processes such as protein sorting, zymogen activation, receptor-mediated endocytosis, and synaptic vesicle proton gradient generation. The ATP6V1B2 protein may include a cytoplasmic V1 domain and a transmembrane V0 domain. Human ATP6V1B2 has an accession number in GenBank of 526. Human ATP6V1B2 may have an accession number in UniProt of P21281.

The term "expression level" generally refers to the protein, RNA or mRNA level of specific related genes. The expression level of the specific related gene (e.g., human ATP6V1B2 gene) can be determined using any method known in the art. In the present application, the "expression" generally refers to a process of transforming gene-encoded information into a structure that is present in and manipulated in a cell. For example, it may include reverse transcription and amplification analysis (e.g., PCR, connected RT-PCR or quantitative RT-PCT), hybridization analysis, RNA Northern blotting, dot blotting, in-situ hybridization, gel electrophoresis, capillary electrophoresis, column chromatography, western blotting, immunohistochemistry, immunostaining or mass spectrometry. Analysis can be performed directly on biological samples or on proteins/nucleic acids isolated from samples.

The term "activity" generally refers to any activity associated with a specific protein. In the present application, the activity may include, for example, any activity associated with the ATP6V1B2 protein. The activity may include protease-associated enzymatic activity. In some cases, the activity may include biological activity. In some cases, the activity may include binding of the protein to a receptor, for example, the binding may produce a measurable downstream effect. In the present application, the activity may include any activity that can be attributed to the protein by those skilled in the art.

The term "proton pump modulator" generally refers to a reagent capable of modulating the activity and/or function of a proton pump.

The term "proton pump agonist" generally refers to a reagent that activates a proton pump. For example, the proton pump agonist can increase the activity of a hydrogen ion pump.

The term "vesicle-type proton pump agonist" generally refers to a reagent that acts to increase the activity of a vesicle-type proton pump. Vesicle-type or bubble-type adenosine triphosphate (V-ATPase) is an ATP-driven proton pump composed of a cytoplasm V1 complex for ATP hydrolysis and a membrane-embedded Vo complex for proton transfer. The vesicle-type proton pump may play an important role in the acidification of vesicles, organelles and extracellular environments within eukaryotic cells.

The term "proton pump-associated protein" generally refers to an associated protein that encodes and/or expresses a proton pump. The proton pump may be a protein that actively transports hydrogen ions on a biological membrane against the electrochemical potential difference of hydrogen ions on both sides of the membrane. The proton pump may include a Na-K pump, a Ca²⁺ pump, an H⁺-ATP pump and an H⁺ pyrophosphoric acid pump.

The term "learning ability" generally refers to all the abilities related to or required for learning/cognitive processes. The learning ability can include the ability to obtain new information, knowledge and/or skills through processes including experiencing, learning or accepting training, etc.. The learning ability can include imagination ability, attention ability, perceptual observation ability, reading ability, analysis ability, manipulation ability, adaption ability, summarization ability, problem solving ability or combinations thereof.

The term "cognitive ability" generally refers to the ability to process information through perception. The cognitive ability can include the ability to grasp the composition of an object, the relationship of performance to other objects, the power of development, the direction of development and the basic rule.

The term "motor ability" generally refers to the ability to participate in sports and training. The motor ability may include aerobic motor ability, muscle strength, body flexibility, balance ability and reaction ability. The motor ability can be a comprehensive representation of various factors such as physical form, quality, skills and psychological ability.

The term "memory ability" generally refers to the ability to remember, maintain, re-recognize and reproduce the content and experience reflected by an objective object. The memory ability may include sensory memory ability, short-term memory ability and long-term memory ability.

The term "spatial exploration ability" generally refers to the ability to explore the shape and/or position of an object. The spatial exploration ability includes observation, thinking, imagination, cognition and/or exploration of the shape and/or position of an object.

The term "evaluation score of learning ability" generally refers to a quantitative evaluation score of the learning ability of a subject. The evaluation score of learning ability can be obtained by attention/performance function evaluation (e.g. Wester Memory test), language ability evaluation (e.g. language screening test (LAST)), view space and structural ability evaluation (e.g. visual motor integration test, Hooper visual tissue test, item piecing test, graphic arrangement test, and clock test), operational ability evaluation, daily function evaluation (e.g. disability assessment for dementia (DAD)) and/or neuropsychiatric scale. In some cases, the evaluation score of learning ability may be obtained by mini-mental state examination (MMSE), Montreal cognitive assessment (MoCA), Alzheimer disease assessment scale-cog (ADAS-cog) and clinical dementia rating scale (CDR) detection.

The term "new object cognitive test" generally refers to a test to enable an animal (e.g., a mouse) to identify an object in a specific space so as to detect the time for the animal to learn to identify a new object. In some cases, the new object cognitive test can refer to the test methods described in Ennaceur et al., Behav Brain Res 80 9-25, 1996. For example, the new object cognitive test may include the following steps: placing two identical objects in a fixed-volume container, placing a mouse in the container to identify the two objects, replacing one of the two objects in the same container with a new object having different shape on the next day, and then measuring the mouse search time for the new object.

The term "water maze test" generally refers to a test to force an animal (e.g., a mouse) to swim, thereby learning to find a platform hidden in water. The water maze test (e.g., Morris water maze) can test the mouse ability to learn and/or memory spatial positional and directional senses. The water maze test may also include acquisition training, probe training, alignment training, or alignment exploration training. If the time required for the animal (e.g., the mouse) to enter water to find the platform is shorter, the distance of movement during this process is about shorter, the evaluation score of the animal (e.g., the mouse) learning ability is correspondingly about higher. The water maze evaluation test can be an important test to evaluate learning ability.

The term "neurodegenerative disease" generally refers to cognitive impairment such as dementia caused by gradual loss of neuronal structure and function, including neuronal death and glial cell balance. In some cases, age (e.g., Alzheimer Disease (AD), Parkinsons Disease (PD)) or gene mutations affecting CNS cell function (e.g., Huntington's chorea, premature AD or PD, Amyotrophic Lateral Sclerosis (ALS)) may cause the neurodegenerative disease. The neurodegenerative disease may have a change and/or condition selected from the group consisting of: protein misfolding and aggregation; neuroinflammation (e.g., CNS inflammation occurring under signal stimulation of toxic stimulation (e.g., protein aggregation), infection, traumatic injury or autoimmunity); change in cellular signal transduction; acquired aging/cell death (e.g., interrupted apoptosis signal transduction, mitochondrial dysfunction, autophagy impairment and stress/inflammation activation of necrotic bodies); and motor cell impairment and epigenetic changes.

The term "Alzheimer disease" generally refers to presenile dementia, and senile dementia, and it is a neurodegenerative disease that is slow in progression and deteriorates over time. The most common early symptoms are loss of short-term memory (difficult to remember the event occurred recently); and when the disease progresses gradually, it may develop gradually at least one of the following symptoms: language disorders, directional disorders (e.g., easy to lose the way), emotional instability, loss of motivation, inability to self-care and behavioral problems. The true cause of Alzheimer disease is still unknown up to now, and its progression may be associated with fibrous amyloid plaque deposition and Tau protein in the brain. There is no treatment that can prevent or reverse the course of disease, but only methods or permissions to temporarily alleviate or ameliorate the symptoms are available.

The term "mild cognitive impairment (MCI)" generally refers to an intermediate clinical state between normal cognition and cognitive impairment. In some cases, the MCI may include cognitive impairment that meets the dementia criteria but to an extent exceeding normal aging. MCI may be diverse in clinical manifestations, etiology, prognosis, and morbidity. In some cases, MCI may be a pathological stage of Alzheimer disease. Certain forms of cognitive impairment may be considered as an early manifestation of neurodegenerative disease, ultimately causing dementia.

The term "normal aging-induced cognitive impairment" generally refers to cognitive impairment due to normal aging. For example, the normal aging-induced cognitive impairment may manifest as: memory loss, confusion in the location of familiar places, longer time to complete daily work than usual, or changes in mood and character.

The term "lews Body Dementia (LBD)" generally refers to Lewy Body Detmentia. Lewy Body Detmentia is characterized by abnormal accumulation of proteins into lumps called Lewy Body. Lewy Body Dementia results in gradual decline in cardiac intelligence. Lewy Body Detmentia patients may have a hallucination and change in alertness and attention. Other effects include muscle stiffness, slow movement, difficulty in walking, and trembling. Patients with the Lewy Body in the brain may also have plaque and tangles associated with Alzheimer disease.

The term "frontotemporal dementia" generally refers to pick disease, which is a rare disease that a tau protein only affects the progressiveness of the frontal and temporal lobes of the brain. Frontotemporal dementia patients are difficult in higher levels of reasoning, expression, perception, and language memory formation. The frontal and temporal lobes of the brain of frontotemporal dementia patients may atrophy over time.

The term "vascular dementia" generally refers to problems in reasoning, judgment, and memory due to damage to brain blood flow. For example, the vascular dementia may include dementia due to factors at risk of heart disease and stroke, such as hypertension and hypercholesterolemia.

The term "multi-infarct" generally refers to non-cortical small infarcts caused by a single perforating branch occlusion of large cerebral artery. The multi-infarct may be a particular type of cerebral infarction, also known as ischemic stroke. The multi-infarct may manifest as: paraesthesia, aphasia, dyskinesia, slowness of action, awkwardness (particularly more difficult in fine actions such as writing).

The term "Parkinson's disease" generally refers to a progressive neurodegenerative disease. Clinical features of the Parkinson's Disease (PD) may include motor symptoms (e.g., tremor, slowness of motion, myotonia, and postural instability), as well as neuropsychiatric and other non-motor manifestations. For example, the non-motor manifestations may include cognitive dysfunction and dementia, mood disorders (e.g., depression, anxiety, apathy), and sleep disorders.

The term "AIDS" generally refers to acquired immunodeficiency syndrome (AIDS). Clinical manifestations of AIDS include changes in memory, concentration, attention, and motor skills. In some cases, AIDS patients may experience cognitive impairments, for example, about 50% of infected persons may further develop into HIV-associated neurocognitive dysfunction (HAND).

The term "Creutzfeldt-Jakob disease (CJD)" generally refers to an infectious spongiform encephalopathy occurring in humans. CJD is a disease caused by prion infection. CJD patients may manifest as: paradoxical behavior, blurred consciousness, loss of appetite and weight, depression, few patients with visual or auditory abnormalities; and during progression, progressive deterioration of the nervous system (e.g. dysesthesia, language disorder and aphasia).

The term "Multiple Sclerosis (MS)" generally refers to a demyelinating neuropathy. The MS patients suffer from damage to the insulating material (i.e. myelin sheath) on the surface of nerve cells in their brain or spinal cord, impaired signal transduction of the nervous system, which may lead to a series of possible symptoms affecting the activity, mind, and even mental state of the patient. These symptoms may include diplopia, unilateral vision impairment, muscle weakness, sensory retardation, or dysordination.

The term "Amyotrophic Lateral Sclerosis (ALS)" generally refers to the symptoms of amyotrophic lateral sclerosis, and motor neuron disease, and is a progressive and fatal neurodegenerative disease. A few ALS patients may suffer from frontotemporal dementia. Some ALS patients suffer from degeneration in sense, vision, touch, smell and taste, and a very few patients suffering from amyotrophic lateral sclerosis may suffer from dementia at the same time.

The term "Huntington's Disease (HD)" is Huntington's chorea, generally a genetic disease that may cause brain cell death. HD patients may suffer from more significant body movement disharmony, with gradual deterioration of ability until movement becomes difficult, unable to speak, as the disease progresses. Cardiac intelligence generally develops to dementia.

The term "aged stage" generally refers to an aging stage of a subject. For example, humans in the aged stage may be more than 60 years old, more than 70 years old or more than 75 years old; and mice in the aged stage may be more than 10 months old, for example more than 13 months old or more than 18 months old. In some cases, the subject of the aged stage may have one or more symptoms of learning deficits, memory disorders, memory deficits and/or brain dysfunction.

The term "variant" generally refers to a polypeptide including an amino acid sequence that differs from the amino acid sequence of a parent or reference polypeptide (e.g. wild-type polypeptide) by at least one amino acid residue. In the present application, the variant may have a higher (e.g. at least 80%) homology with the parent or reference polypeptide. The homology may include the similarity or identity of the sequence. In the present application, the homology can be determined using standard techniques known in the art (see, for example, Smith and Waterman, Adv. Appl .Math. Advances in Applied Mathematics); and the percentage of identity common to polynucleotide or polypeptide sequences is determined by direct comparison of sequence information between molecules, the comparison being made by sequence alignment and identity determination using methods known in the art. Examples of algorithms suitable for determining sequence similarity are BLAST algorithms (see, Altschure et al., Jay Mohr. Bio. Journal of Molecular Biology, 215:403-410 [1990]). Software for BLAST analysis can be obtained by the National Center of Biotechnology Information (NCBI) in publication.

The term "transmitter release" generally refers to neurotransmitter release, namely a process that a neuron acts on another neuron by releasing neurotransmitters wrapped in a vesicle to a synaptic space so as to transfer information. In the process of the transmitter release, the basic structure synapse of a neural loop can be involved. In some cases, the transmitter release can be called synaptic transmission. The method of the transmitter release may include synchronous release, asynchronous release and spontaneous release.

The term "release frequency" generally refers to the release frequency of an action potential. The action potential may refer to a process of quickly and reversibly reversing and restoring potentials on two sides of a primary membrane generated on the basis of a resting potential when excitable cells are stimulated. The action potential can consist of a peak potential and a post potential which respectively correspond to a depolarisation process and a hyperpolarization process. The release of the action potential may have the characteristics of pulses. In some cases, the release frequency of the pulses is the ratio of the number of the released pulses to the time.

In the present application, the term "neuron" generally refers to nerve cells which are main functional units of the nervous system. The neuron can consist of a cell body, a protuberant axon thereof and one or more dendritic processes. The neuron can transfer information to other neurons or cells by releasing the neurotransmitters at the synapse.

The term "multimer" generally refers to molecules having two or more polypeptide chains that are covalently, non-covalently, or simultaneously associated by covalent and non-covalent interactions. The multimer may include a dimer.

The term "homodimers" generally refers to molecules formed from two identical monomers. The two identical monomers may be aggregated, complexed, or associated with each other by covalent and/or non-covalent interactions.

The term "sulflrydryl blocking" generally refers that free sulfhydryl groups is blocked to make it difficult to form intramolecular and/or intermolecular disulfide bonds. In the present application, the sulfhydryl blocking may occur on cysteine residues. The sulfhydryl blocking may make it impossible to form disulfide bonds between cysteine residues of a protein to prevent crosslinking or modification of the protein. The sulfhydryl blocking can be achieved by a blocking reagent, which may be a reducing reagent. The blocking reagent may include Dithiothreitol (DTT), β-mercaptoethanol (BME), and tris(2-carboxyethyl) phosphonate (TCEP•HCl).

The term "serine phosphorylation" generally refers to phosphorylation modification that occurs on serine residues. The serine phosphorylation may be a process of transferring phosphate groups of a donor (e.g., ATP or GTP) onto serine residues. The serine phosphorylation may be by means of a protein kinase. The serine phosphorylation may result in a change in protein activity.

The term "about" usually refers to a numerical range of 20% more or less than a specific value. For example, "about X" includes a range of values that are ±20%, ±10%, ±5%, ±2%, ±1%, ±0.5%, ±0.2%, or ±0.1% of X, where X is a numeric value.

### DETAILED DESCRIPTION of THE PRESENT INVENTION

In one aspect, the present application provides use of a proton pump modulator in preparing a reagent, and the reagent is used for improving learning ability.

In another aspect, the present application provides use of a proton pump modulator in preparing a reagent, and the reagent is used for treating cognitive impairment.

In another aspect, the present application provides use of a proton pump modulator in preparing a reagent, and the reagent is used for treating neurodegenerative diseases.

In another aspect, the present application provides use of an ATP6V1B2 modulator in preparing a reagent, and the reagent is used for improving learning ability.

In another aspect, the present application provides use of an ATP6V1B2 modulator in preparing a reagent, the reagent is used for treating cognitive impairment.

In another aspect, the present application provides use of an ATP6V1B2 modulator in preparing a reagent, the reagent is used for treating neurodegenerative diseases.

In the present application, when the expression level and/or activity of ATP6V1B2 in a subject is increased, the learning ability of the subject can be significantly improved (for example, the cognitive ability, motor ability, memory ability and/or spatial exploration ability can be significantly improved as compared with the expression level and/or activity of ATP6V1B2 in the subject before being improved). Therefore, ATP6V1B2 can be used as a potential target for improving the learning ability. For example, ATP6V1B2 can be used as a potential target for treating neurodegenerative diseases (for example, Alzheimer disease) and/or cognitive impairments.

### Improvement of expression level and/or activity of proton pump-associated protein and ATP6V1B2

In the present application, the reagent can improve the expression level and/or activity of ATP6V1B2 in the subject.

In the present application, the proton pump modulator can improve the expression level and/or activity of ATP6V1B2 in the subject.

In the present application, the ATP6V1B2 modulator can improve the expression level and/or activity of ATP6V1B2 in the subject.

In the present application, the expression level of the ATP6V1B2 and/or the functional active fragment thereof may include the expression level of an ATP6V1B2 gene, the transcription level of the ATP6V1B2 gene, and/or the expression level of ATP6V1B2 protein. For example, the expression level may include the amount of polynucleotide, mRNA or amino acid product or protein of a specific gene (for example, human ATP6V1B2 gene). The expression level may include the amount of polynucleotide transcribed from a specific gene (for example, human ATP6V1B2 gene), translated protein or a fragment of a post-translational modified protein.

In the present application, the improvement may include that the expression level of ATP6V1B2 is improved by at least about 10% as compared with the expression level and/or activity of original ATP6V1B2 in the subject. For example, it can be improved by at least about 15%, at least about 20%, at least about 25%, at least about 30%, at least about 35%, at least about 40%, at least about 45%, at least about 50%, at least about 55%, at least About 60%, at least about 65%, at least about 70%, at least about 75%, at least about 80%, at least about 85%, at least about 90%, at least about 95%, at least about 100%, at least about 200%, at least about 500% or more.

In the present application, the expression level of ATP6V1B2 can be measured by utilizing a substance selected from the following groups: a primer capable of specifically amplifying the ATP6V1B2 gene, a nucleic acid molecule specifically bound to the ATP6V1B2 gene, a nucleic acid molecule specifically bound to ATP6V1B2 protein, a small molecule specifically bound to the ATP6V1B2 protein, a probe specifically bound to the ATP6V1B2 protein, and polypeptides specifically bound to the ATP6V1B2 protein.

In the present application, the expression level of the ATP6V1B2 can be measured by implementing a test selected from the following groups: reverse transcription and amplification analysis (e.g. PCR, connected RT-PCR or quantitative RT-PCT), hybridization analysis, RNA Northern blotting, dot blotting, in-situ hybridization, gel electrophoresis, capillary electrophoresis, column chromatography, western blotting, immunohistochemistry, immunostaining or mass spectrometry. For example, the expression level of ATP6V1B2 according to the present application can be measured through qPCR, qRT-PCR, northern hybridization, western hybridization and/or ELISA detection. The expression level of ATP6V1B2 can also be measured by directly analyzing a biological sample or analyzing protein/nucleic acid separated from the sample.

In the present application, the ATP6V1B2 can be derived from human or mouse.

In some embodiments, the ATP6V1B2 may include the amino acid sequence as set forth in SEQ ID NO. 8 or 16.

In the present application, the functional active fragment of the ATP6V1B2 has the capacity of specifically binding to the amino acid sequence as set forth in SEQ ID NO. 5. For example, the functional active fragment of the ATP6V1B2 includes a truncation of the ATP6V1B2. For example, the functional active fragment of the ATP6V1B2 may include at least part of sequence of the 288th-512th amino acid sequence of human ATP6V1B2 protein. For example, the functional active fragment of the ATP6V1B2 may include at least part of sequence of the 288th-512th amino acid sequence of mouse ATP6V1B2 protein.

In the present application, the ATP6V1B2 and/or the functional active fragment thereof may include the amino acid sequence as set forth in any one of SEQ ID NO. 8, 10-11, 16.

In the present application, the nucleic acid sequence encoding the ATP6V1B2 and/or the functional active fragment thereof may include the nucleic acid sequence as set forth in SEQ ID NO: 9 or 17.

In the present application, the activity of the ATP6V1B2 may include the biological activity of ATP6V1B2 protein and/or the functional active fragment thereof (e.g., may include the resulting measurable downstream effect). For example, the activity of the ATP6V1B2 may include improving the expression levels and/or activity of the proton pump-associated protein.

In the present application, the improvement may include that the activity of the ATP6V1B2 is improved by at least about 10% as compared with the activity of original ATP6V1B2 in the subject. For example, it can be improved by at least about 15%, at least about 20%, at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 95%, at least about 100%, at least about 200%, at least about 500% or more.

In the present application, the reagent can improve the expression level and/or activity of proton pump-associated protein in a subject.

In the present application, the proton pump modulator can improve the expression level and/or activity of proton pump-associated protein in the subject.

In the present application, the ATP6V1B2 modulator can improve the expression level and/or activity of the proton pump-associated protein in the subject.

In the present application, the expression level of the proton pump-associated protein may include the expression level of a gene encoding the proton pump-associated protein, the transcription level of the gene encoding the proton pump-associated protein and/or the expression level of the proton pump-associated protein. For example, the expression levels may include the amount of polynucleotides, mRNAs, or amino acid products or proteins of specific genes (e.g., genes encoding proton pump-associated proteins). The expression levels may include the amount of transcribed polynucleotides, translated proteins, or fragments of post-translational modified proteins of specific genes (e.g., genes encoding proton pump-associated proteins).

In the present application, the improvement includes that the expression level of the proton pump-associated protein is improved by at least about 10% as compared with the expression level of original proton pump-associated protein in the subject. For example, it can be improved by at least about 15%, at least about 20%, at least about 25%, at least about 30%, at least about 35%, at least about 40%, at least about 45%, at least about 50%, at least about 55%, at least About 60%, at least about 65%, at least about 70%, at least about 75%, at least about 80%, at least about 85%, at least about 90%, at least about 95%, at least about 100%, at least about 200%, at least about 500% or more.

In the present application, the expression level of the proton pump-associated protein is measured by a substance selected from the following groups: a primer capable of specifically amplifying the gene encoding the proton pump-associated protein, a nucleic acid molecule specifically bound to the gene encoding the proton pump-associated protein, a nucleic acid molecule specifically bound to the proton pump-associated protein, a small molecule specifically bound to the proton pump-associated protein, a probe specifically bound to the proton pump-associated protein, and polypeptide specifically bound to the proton pump-associated protein.

In the present application, the expression level of the proton pump-associated protein can be measured by implementing a test selected from the following groups: reverse transcription and amplification analysis (e.g. PCR, connected RT-PCR or quantitative RT-PCT), hybridization analysis, RNA Northern blotting, dot blotting, in-situ hybridization, gel electrophoresis, capillary electrophoresis, column chromatography, western blotting, immunohistochemistry, immunostaining or mass spectrometry. For example, the expression level of the proton pump related protein according to the present application can be measured through qPCR, qRT-PCR, northern hybridization, western hybridization and/or ELISA detection. The expression level of the proton pump-associated protein can also be measured by directly analyzing a biological sample or analyzing protein/nucleic acid separated from the sample.

In the present application, the proton pump-associated proteins may include NADH dehydrogenase, coenzyme Q, succinate-coenzyme Q reductase, cytochrome c, and/or coenzyme Q-cytochrome c reductase.

In the present application, the activity of the proton pump-associated protein can be the biological activity of the proton pump-associated protein.

The activity of the proton pump-associated protein can be measured through the activity level of a hydrogen/potassium adenosine triphosphate enzyme system (also known as hydrogen/potassium ion ATPase, i.e., H⁺/K⁺ ATPase); and/or activity level of an H₂ receptor. In the present application, the improvement of the activity of the proton pump-related protein may include that the activity of the proton pump-related protein is improved by at least about 10% as compared with the activity of the original proton pump-related protein in the subject. For example, it can be improved by at least about 15%, at least about 20%, at least about 25%, at least about 30%, at least about 35%, at least about 40%, at least about 45%, at least about 50%, at least about 55%, at least About 60%, at least about 65%, at least about 70%, at least about 75%, at least about 80%, at least about 85%, at least about 90%, at least about 95%, at least about 100%, at least about 200%, at least about 500% or more.

### Learning ability

In the present application, the learning ability may include all abilities that are related to or required for learning/cognitive processes. For example, the learning ability may include cognitive may, motor may, memory may, and/or spatial exploration may.

In the present application, the improvement of the learning ability includes that compared with an evaluation score of original learning ability of the subject, the evaluation score of the improved learning ability of the subject is increased by at least about 50%. For example, it can be increased by at least about 55%, at least about 60%, at least about 65%, at least about 70%, at least about 75%, at least about 80%, at least about 85%, at least about 90%, at least about 95%, at least about 100%, at least about 200%, at least about 500% or more.

In the present application, the evaluation score of learning ability can be obtained by attention/performance function evaluation (e.g. Wester Memory test), language ability evaluation (e.g. language screening test (LAST)), view space and structural ability evaluation (e.g. visual motor integration test, Hooper visual tissue test, item piecing test, graphic arrangement test, and clock test), operational ability evaluation, daily function evaluation (e.g. disability assessment for dementia (DAD)) and/or neuropsychiatric scale. For example, the evaluation score of learning ability can be measured by a test selected from the group consisting of: a new object cognitive test and a water maze test.

In the present application, the new object cognitive test can evaluate the cognitive ability, motor ability and/or spatial exploration ability. In the present application, the new object cognitive test can enable a subject (such as a mouse) to explore and learn an object with a specific shape in a fixed container, and distinguish a new object which appears in the fixed container and is different from the previous object in shape after several days according to the memory obtained by learning. If the time for distinguishing the new object with the different shape of the mouse after several days is shorter, the learning ability of the mouse is correspondingly higher. The water maze evaluation test can be an important experiment for evaluating the learning ability of the subject.

In the present application, the water maze test can evaluate the memory ability, motor ability and/or spatial exploration ability. In the present application, the water maze test (such as a Morris water maze) is to force the subject (such as a mouse) to swim, thereby learning to find a platform hidden in water, so as to test the spatial exploration ability and/or memory ability of the mouse to the space position sense and direction sense. The water maze test can also include acquisition training, exploration training, alignment training or alignment exploration training. If the time required for the mouse to enter water to find the platform is shorter, the distance of movement during this period is shorter, and the learning ability of the mouse is correspondingly higher. The water maze evaluation test can be an important test to evaluate the learning ability of the subject.

### Subjects and indications

In the present application, the subject may include mammals. For example, the subject may include a rodent and/or a primate, for example, the subject may include human.

In the present application, the subject may include a patient with non-cognitive impairment and/or a patient with non-neurodegenerative disease. For example, the subject may be normal and/or healthy human. For example, the subject may have needs and/or desires to further improve their ability to learn.

In the present application, the neurodegenerative disease includes acute neurodegenerative disease and chronic neurodegenerative disease. For example, the neurodegenerative disease includes neurodegenerative disease caused by neuronal death and glial cell homeostasis, neurodegenerative disease caused by aging, neurodegenerative disease caused by influence on the CNS cell function, neurodegenerative disease caused by abnormal communication between cells, and/or neurodegenerative disease caused by damage to cell motor.

In the present application, the subject may include a patient with neurodegenerative disease. For example, the subject may include a patient with Alzheimer disease. For example, patient with Alzheimer disease can be in the pre, mild, middle, or late stages of Alzheimer disease.

In the present application, the cognitive impairment may include mild cognitive impairment (MCI), middle cognitive impairment and late cognitive impairment. For example, the cognitive impairments may include cognitive impairments caused by normal aging, Lews Body Dementia (LBD), frontotemporal dementia and/or vascular dementia. For example, the diseases induced by cognitive impairment may include Alzheimer disease, multi-infarct, Parkinson's disease, AIDS and/or Creutzfeldt-Jakob disease (CJD).

In the present application, the subject may include a patient with cognitive impairment. For example, the subject may suffer from mild cognitive impairment (MCI) (e.g., loss of short-term memory, difficult to express or understand abstract things, precarious emotion or behavior, difficult to learn new things and follow complex instructions, hypojudgment and/or need of other people to remind in basic self-care), medium cognitive impairment (e.g., confusion of long-term memory and real-time memory, word inadequacy, behavioral character transition or instable emotion and/or need of other people to assist in self-care) or late cognitive impairment (e.g., memory impairment, decline in physical activity and mental condition, unable to effectively express or communicate, unable to self-care and/or biological clock confusion). In the present invention, the subject may suffer from a disease which can induce the cognitive impairment. For example, the subject may suffer from Alzheimer disease, multi-infarct, Parkinsons disease, AIDS and/or Creutzfeldt-Jakob disease (CJD).

In the present application, the subject may be in the aged stage. For example, the subject has exhibited cognitive impairment due to normal aging. For example, the subject has exhibited symptoms of early cognitive impairment (MCI). For example, the subject has exhibited symptoms of neurodegenerative diseases (such as Alzheimer disease).

### Polypeptides or variants thereof, reagents and pharmaceutical composition and use thereof

In the present application, the proton pump modulator may include a proton pump agonist.

In the present application, the proton pump modulator may include a vesicle-type proton pump agonist.

In the present application, the proton pump modulator may include an agonist of the ATP6V1B2 protein.

In the present application, the proton pump modulator can be bound to the ATP6V1 B2 protein. For example, the proton pump modulator may include a ligand for the ATP6V1B2 protein. For example, the proton pump modulator may include the agonist of the ATP6V1B2 protein. For example, the proton pump modulator can be specifically bound to a specific location (e.g., a specific epitope) in the ATP6V1B2 protein.

In the present application, the proton pump modulator may include nucleic acid or a variant thereof, polypeptide or a variant thereof, and/or small molecule .

In the present application, the proton pump modulator can increase the synaptic neurotransmitter release.

In the present application, the increase includes that at least about 10% is increased as compared with the original synaptic neurotransmitter release level in the subject. For example, it can be increased by at least about 20%, at least about 50%, at least about 60%, at least about 65%, at least about 70%, at least about 75%, at least about 80%, at least about 85%, at least about 90%, at least about 95%, at least about 100%, at least about 200%, at least about 500% or more.

In the present application, the proton pump modulator can increase the release frequency of excitatory postsynaptic current.

In the present application, the increase includes that at least about 10% is increased as compared with the release frequency level of the excitatory postsynaptic current in the subject. For example, it can be increased by at least about 20%, at least about 50%, at least about 60%, at least about 65%, at least about 70%, at least about 75%, at least about 80%, at least about 85%, at least about 90%, at least about 95%, at least about 100%, at least about 200%, at least about 500% or more.

In the present application, the ATP6V1B2 modulator may include an ATP6V1B2 agonist.

In the present application, the ATP6V1B2 can be bound to the ATP6V1B2 protein. For example, the ATP6V1B2 modulator may include a ligand for the ATP6V1B2 protein. For example, the ATP6V1B2 modulator may include the agonist of the ATP6V1B2 protein. For example, the ATP6V1B2 modulator can be specifically bound to a specific location (e.g., a specific epitope) in the ATP6V1B2 protein.

In the present application, the ATP6V1B2 modulator may include a proton pump agonist, for example, it may include a vesicle-type proton pump agonist.

In the present application, the ATP6V1B2 modulator may include nucleic acid or a variant thereof, polypeptide or a variant thereof, and/or small molecule .

In the present application, the ATP6V1B2 modulator can increase the synaptic neurotransmitter release.

In the present application, the increase includes that at least about 10% is increased as compared with the original synaptic neurotransmitter release level in the subject. For example, it can be increased by at least about 20%, at least about 50%, at least about 60%, at least about 65%, at least about 70%, at least about 75%, at least about 80%, at least about 85%, at least about 90%, at least about 95%, at least about 100%, at least about 200%, at least about 500% or more.

In the present application, the ATP6V1B2 modulator can increase the release frequency of excitatory postsynaptic current.

In the present application, the increase includes that at least about 10% is increased as compared with the release frequency level of the excitatory postsynaptic current in the subject. For example, it can be increased by at least about 20%, at least about 50%, at least about 60%, at least about 65%, at least about 70%, at least about 75%, at least about 80%, at least about 85%, at least about 90%, at least about 95%, at least about 100%, at least about 200%, at least about 500% or more.

In another aspect, the present application provides polypeptide capable of modulating expression and/or activity of ATP6V1B2.

In the present application, the polypeptide can increase the synaptic neurotransmitter release. In the present application, the increase includes that at least about 10% is increased as compared with the original synaptic neurotransmitter release level in the subject. For example, it can be increased by at least about 20%, at least about 50%, at least about 60%, at least about 65%, at least about 70%, at least about 75%, at least about 80%, at least about 85%, at least about 90%, at least about 95%, at least about 100%, at least about 200%, at least about 500% or more.

In the present application, the polypeptide can increase the release frequency of excitatory postsynaptic current. In the present application, the increase includes that at least about 10% is increased as compared with the release frequency level of the excitatory postsynaptic current in the subject. For example, it can be increased by at least about 20%, at least about 50%, at least about 60%, at least about 65%, at least about 70%, at least about 75%, at least about 80%, at least about 85%, at least about 90%, at least about 95%, at least about 100%, at least about 200%, at least about 500% or more.

In the present application, the polypeptide or variant thereof can be specifically bound to the ATP6V1B2 protein. For example, the polypeptide or variant thereof may have a "ligand-acceptor" relationship with the ATP6V1B2 protein.

In the present application, the polypeptide or variant thereof can be bound to at least part of sequence of the 288th to 512th amino acid sequence of mouse ATP6V1B2 protein. In the present application, the polypeptide or variant thereof can be bound to at least part of sequence of the 288th to 512th amino acid sequence of human ATP6V1B2 protein.

In the present application, the polypeptide or variant thereof may include molecules of monomers (amino acids) that are linearly linked by an amide bond (also known as a polypeptide bond). The polypeptide may include any chain having two or more amino acids, and does not refer to a product of a particular length. The variant may include a polypeptide with substitution, addition, or deletion of one or more amino acid on the polypeptide basis. The variant may include a product having modifications on the polypeptide basis, such as glycosylation, acetylation, phosphorylation, acylation, derivatization by known protective/blocking groups, proteolytic cleavage, and/or modification by non-naturally occurring amino acids.

In the present application, the polypeptide or variant thereof can improve the expression level and/or activity of the ATP6V1B2 in the subject. The expression level of the ATP6V1B2 comprises the expression level of an ATP6V1B2 gene, the transcription level of the ATP6V1B2 gene and/or the expression level of ATP6V1B2 protein. In the present application, the improvement includes that the expression level and/or activity of ATP6V1B2 is improved by at least about 10% as compared with the expression level and/or activity of original ATP6V1B2 in the subject. For example, it can be increased by at least about 20%, at least about 50%, at least about 60%, at least about 65%, at least about 70%, at least about 75%, at least about 80%, at least about 85%, at least about 90%, at least about 95%, at least about 100%, at least about 200%, at least about 500% or more.

In the present application, the polypeptide may include the amino acid sequence as set forth in SEQ ID NO. 5.

In the present application, the polypeptide may include the amino acid sequence as set forth in any one of SEQ ID NO. 1 and 3.

In the present application, the polypeptide or variant thereof may form a multimer. For example, homodimer can be formed. In the present application, the homodimer may still have the ability to bind to the ATP6V1B2 protein.

The homodimer can be a tandem structure of two identical polypeptides. For example, the homodimer may include the amino acid sequence as set forth in SEQ ID NO. 18.

In the present application, cysteines in the amino acid sequence of the polypeptide or variant thereof may not have a sulfhydryl-blocking modification. For example, if the cysteine residue in the amino acid sequence of the polypeptide or variant thereof is blocked by a sulfhydryl group, it may lose its ability to bind to the ATP6V1B2 protein.

In the present application, serine in the amino acid sequence of the polypeptide or variant thereof may not have a phosphorylation modification. For example, if the serine residue in the amino acid sequence of the polypeptide or variant thereof is phosphorylated, it may lose its ability to bind to the ATP6V1B2 protein.

In the present application, the preparation (e.g., the polypeptide or variant thereof) may improve the learning ability of the subject (e.g., significantly improve the assessment score of the learning ability of the subject as compared with that before administration of the polypeptide or variant thereof).

In the present application, the preparation (such as polypeptide or variant thereof) can regulate the expression quantity and/or activity of genes related to learning ability in the subject. In the present application, the genes related to learning ability may include an APP gene. The APP gene is an amyloid precursor protein gene which may be related to Alzheimer disease, such as Familial Alzheimer disease (FAD). In addition, the learning ability-related gene may include presenilin1 gene (PSEN1) and presenilin2 gene (PSEN2). For example, the learning ability-related gene may include a gene encoding a glutamate receptor. For example, the glutamate receptor may include an N-methyl-D-aspartate receptor (NMDAR), an alginic acid receptor (KAR), and a α-amino-3-hydroxy-5-methyl-4-isoxazole receptor (AMPAR). For example, the learning ability-related gene may include a gene encoding an action receptor for memantine (e.g., 1-amino-3,5-dimethyladamantane hydrochloride). For example, the action receptor for memantine may include an NMDA receptor. For example, the polypeptide may reduce the expression and/or activity of the above-mentioned gene in the present segment.

In the present application, the preparation according to the present application, the ATP6V1B2 modulator, the proton pump modulator, and/or the polypeptide according to the present application may be used as potential drugs to improve learning ability. The preparation according to the present application, the ATP6V1B2 modulator, the proton pump modulator, and/or the polypeptide according to the present application may be used as potential drugs to treat cognitive impairment and/or neurodegenerative diseases.

In another aspect, the present application provides a pharmaceutical combination which includes the reagent according to the present application.

The pharmaceutical combination may include a pharmaceutical product for pharmaceutical purposes (e.g., improving learning ability, treating cognitive impairment, and/or treating neurodegenerative diseases (such as Alzheimer disease)).

In another aspect, the present application provides a pharmaceutical composition, including the reagent according to the present application and a pharmaceutically acceptable carrier.

The pharmaceutical composition may be a composition including one or more active ingredients (such as the preparation according to the present application) and one or more inert components; and any product resulting directly or indirectly from the combination, compounding or aggregation of any two or more components, or by the dissociation of one or more components, or by other types of reactions or interactions of one or more components.

The pharmaceutically acceptable carrier may include sterile water or non-aqueous solutions, dispersions, suspensions or emulsions, and sterile powders that are used for reconstituting the sterile injectable solution or dispersion immediately prior to use. Examples of suitable water and non-aqueous carriers, diluents, solvents or menstruums may include water, ethanol, polyols (e.g., propylene glycol, and polyethylene glycol), cluster methylcellulose and suitable mixtures thereof, vegetable oils (e.g., olive oil) and injectable organic vinegar such as ethyl oleate.

In another aspect, the present application provides a use of the reagent according to the present application, the pharmaceutical combination according to the present application and/or the pharmaceutical composition according to the present application in preparing drugs for increasing the synaptic neurotransmitter release.

In another aspect, the present application provides a use of the reagent according to the present application, the pharmaceutical combination according to the present application and/or the pharmaceutical composition according to the present application in preparing drugs for improving learning ability.

In another aspect, the present application provides a use of the reagent according to the present application, the pharmaceutical combination according to the present application and/or the pharmaceutical composition according to the present application in preparing drugs for treating cognitive impairment, and/or treating neurodegenerative diseases.

In the present application, the polypeptide according to the present application, the reagent according to the present application, the pharmaceutical combination according to the present application and/or the pharmaceutical composition according to the present application can be formulated to be suitable for oral administration and/or injection administration. In the present application, the polypeptide according to the present application, the reagent according to the present application, the pharmaceutical combination according to the present application and/or the pharmaceutical composition according to the present application can be formulated to be suitable for intravenous injection.

### Drug screening method

In another aspect, the present application provides a method for screening drugs capable of improving learning ability, treating cognitive impairment, and/or treating neurodegenerative diseases of a subject, including the following step: detecting the influence of the candidate drugs on the expression level and/or activity of proton pump-associated protein in the subject; and after the candidate drugs are applied, the expression level and/or activity of the proton pump-associated protein are improved, and the candidate drugs can improve the learning ability, treat cognitive impairment, and/or treat neurodegenerative diseases of the subject.

In another aspect, the present application provides a method for screening drugs capable of improving learning ability, treating cognitive impairment, and/or treating neurodegenerative diseases of a subject, including the following step: detecting the influence of the candidate drugs on the expression level and/or activity of ATP6V1B2 in the subject, wherein after the candidate drugs are applied, the expression level and/or activity of the ATP6V1B2 are improved, and the candidate drugs can improve the learning ability, treat cognitive impairment, and/or treat neurodegenerative diseases of the subject.

In some cases, the candidate drugs may include a reagent capable of modulating the expression level and/or activity of the ATP6V1B2; and in some cases, the candidate drug may include a reagent capable of modulating the expression level and/or activity of the proton pump-associated protein. In some cases, the candidate drug may include a reagent capable of binding the ATP6V1 B2 protein.

In the present application, the administration may include oral and/or injection administration.

In the present application, the administration may include intravenous injection.

The present application also provides an assay kit that can detect the expression level and/or activity of the ATP6V1B2. The assay kit may include an instruction manual that describes the specific steps of how to use the assay kit to detect the expression level and/or activity of the proton pump-related protein and/or ATP6V1B2, and/or how to use the test results to determine whether the candidate drug can improve the learning ability of the subject.

In the present application, the test kit may also include a reagent capable of detecting other targets that can determine whether the candidate drug can improve the learning ability of the subject.

### Non-human animals and offspring thereof or parts thereof

In any aspect according to the present application, the non-human animal is a gene-edited non-human animal, or derived from the gene-edited non-human animal. For example, the non-human animal is a gene-knocked-in non-human animal, or is derived from a gene-knocked-in non-human animal.

For example, the non-human animal is a non-human mammal. In some embodiments, the non-human animal is a rodent or a primate. In some embodiments, the non-human animal is selected from: mice, rats, rabbits, apes, monkeys, pigs, cows, sheep, horses, chickens, dogs, alpacas and cats.

In any aspect according to the present application, the part of the non-human animal or offspring thereof may include organs, tissues and/or cells.

In some embodiments, the part includes body fluid. For example, the body fluid may be selected from the following groups: blood, plasma, serum, urine, sweat, tears, saliva, semen and cerebrospinal fluid. In some embodiments, the plasma includes exosomes. In some embodiments, the part includes brain (e.g., brain tissue) or part of the brain. The part of the brain may include the part selected from the following groups: olfactory bulb, amygdala, basal ganglia, hippocampus, thalamus, hypothalamus, cerebral cortex, medulla oblongata, and cerebellum. In some embodiments, the part includes olfactory mucosa. In some embodiments, the part includes part of the central nervous system, part of the peripheral nervous system, skin tissue, muscle tissue, and/or internal organs. For example, the central nervous system may include spinal cord.

In some embodiments, the part may include cells. The cells are selected from the following groups: primary neurons, microglia, astrocytes, oligodendrocytes, macrophages, perivascular epithelioid cells, B cells, T cells, adult stem cells, NK cells, totipotent stem cells, unipotent stem cells, embryonic stem cells, induced pluripotent stem cells, and gametes. For example, the gametes may include spermatozoa and/or oocytes.

In some embodiments, the part (for example, the organs, tissues or cells) cannot develop into a complete non-human animal.

In some embodiments, the non-human animal and offspring thereof, or the part thereof include the ATP6V1B2 and/or the functional active fragment thereof. For example, the ATP6V1B2 and/or the functional active fragment may be expressed by the animal and offspring thereof, or the cell, or may be derived from another source and subsequently introduced (e.g., injected) into the animal and offspring thereof, or the cell, so that it includes the ATP6V1B2 and/or the functional active fragment thereof.

In some embodiments, the non-human animal and offspring thereof, or part thereof expresses the ATP6V1B2 and/or the functional active fragment thereof.

The expression of ATP6V1B2 may include the expression of ATP6V1B2 genes, transcripts of ATP6V1B2 genes, and/or expression of ATP6V1B2 proteins. For example, the expression may include the expression of polynucleotides, mRNAs, or amino acid products, or proteins of specific genes (e.g., human ATP6V1B2 genes). The expression may include the expression of transcribed polynucleotides, translated proteins, or fragments of post-translational modified proteins of specific genes (e.g., human ATP6V1B2 genes).

The expression of the ATP6V1B2 functional active fragment may include the expression of the functional active fragment gene encoding the ATP6V1B2, the transcript encoding the ATP6V1B2 functional active fragment gene, and/or the expression of the ATP6V1B2 functional active fragment protein. For example, the expression may include the expression of polynucleotides, mRNAs, or amino acid products or proteins of specific genes (e.g., functional active fragments encoding human ATP6V1B2 (e.g., amino acid sequence 288 to 512 thereof)). The expression may include the expression of transcribed polynucleotides, translated proteins, or fragments of post-translational modified proteins of specific genes.

### Preparation method

The present application also provides a method for preparing the non-human animal or part thereof according to the present application, the cell according to the present application, or the tissue according to the present application; and the method includes: enabling the non-human animal or part thereof, the cell or the tissue to include and/or express the ATP6V1B2 and/or the functional active fragment thereof.

In another aspect, the present application further provides a method for preparing a disease model; and the method includes: enabling the non-human animal or part thereof, the cell or the tissue to include and/or express the ATP6V1B2 and/or the functional active fragment thereof.

For example, the method may include: introducing the ATP6V1B2, a functional active fragments thereof, a nucleic acid molecule encoding the ATP6V1B2 and/or a nucleic acid molecule encoding the functional active fragment of the ATP6V1B2 into the non-human animal or part thereof, the cell, the tissue, the cell line and/or the cell culture. The introduction may include, for example, injecting the non-human animal, the part thereof, the tissue or the cell (for example, injecting protein or nucleic acid molecules). For example, the introduction may also include nucleic acid molecule transfection, virus transduction and other means for introducing foreign proteins, foreign nucleic acid molecules and the like into the cell, the tissue or the animal. For example, the transfection may include transfection by adeno-associated viruses.

In some embodiments, the method may include: knocking in the nucleic acid sequence encoding the ATP6V1B2 and/or the functional active fragment thereof in the non-human animal or part thereof, the cell, tissue, cell line, and/or cell culture.

In some embodiments, the method may further include: identifying the modified cells or non-human animals including the knock-in the heterologous nucleic acid sequence.

Donor nucleic acid molecules including the heterologous nucleic acid sequence may also include a 5' homologous arm and a 3' homologous arm. The 5' homologous arm and the 3' homologous arm can be flanked by the heterologous nucleic acid sequence encoding the ATP6V1B2 and/or the functional active fragment thereof. The homologous arm in the donor nucleic acid molecules (e.g., 5' homologous arm or 3' homologous arm) may have sufficient facilitation with endogenous target sites.

In the present application, the homologous arm and the target gene locus (i.e., a homologous gene region or corresponding region within the endogenous ATP6V1B2 gene locus) match or correspond to each other, and the mutual agreement of the two local sequences is sufficient as the basis for the homologous recombination reaction. "Homology" means that the DNA sequence is identical or has some sequence consistency with the corresponding or matching sequence. Sequence agreement between a particular target site and the corresponding homologous arm found in the donor nucleic acid molecules may be any degree of sequence agreement that allows homologous recombination to occur. For example, the degree of sequence identity shared by the homologous arm of the donor nucleic acid molecule(or a fragment thereof) with the target site (or a fragment thereof) can be at least about 50%, at least about 55%, at least about 60%, at least about 65%, at least about 70%, at least about 75%, at least about 80%, at least about 81%, at least about 82%, at least about 83%, at least about 84%, at least about 85%, at least about 86%, at least about 87%, at least about 88%, at least about 89%, at least about 90%, at least about 91%, at least about 92%, at least about 93%, at least about 94%, at least about 95%, at least about 96%, at least about 97%, at least about 98%, at least about 99%, or at least about 100%, in order to perform homologous recombination of the sequence.

### Screening/identification/design methods

For example, the device may include a medical device. For example, medical devices that have been claimed to be effective in improving the learning ability, treating cognitive impairment, and/or treating neurodegenerative diseases of the subjects.

The identification, screening, or design method according to the present application may be vitro, ex vivo, or in vivo methods.

According to the present application, the candidate substance may be a synthetic compound, polypeptide, protein, DNA library or nucleic acid molecules in that library, an animal (e.g., mammalian such as a mouse, rat, pig, cattle, sheep, monkey or human) tissue extract or cell culture supernatant, an extract or culture product of a plant or microorganism, or any mixture of the above.

The cognitive ability, the motor ability, the memory ability, and/or the spatial exploration ability can also be compared between treatment and control groups administered with the candidate drugs, e.g. by performing animal behavior analysis. For example, if significant improvements in the cognitive ability, the motor ability, the memory ability, and/or the spatial exploration ability are observed after treatment with the candidate drugs compared to the control group, the candidate drug may be selected for further study (e.g., as a potential treatment drug to improve the learning ability, treat the cognitive impairment, and/or treat the neurodegenerative diseases of the subjects).

In some cases, the screening method can also be performed using the non-human animal tissue and/or cell according to the present invention, such as neuronal cells, brain regions, or other tissues including/corresponding to AD lesions, or part of the tissue (e.g., olfactory bulbs, amygdala, basal ganglia, hippocampus, thalamus, hypothalamus, cerebral cortex, medulla oblongata, the central nervous system, the peripheral nervous system, spinal cord, cerebellum, skin tissue, muscle tissue, and/or internal organs). For example, the tissue and/or cell can be cultured in vitro or ex vivo, and then, after appropriate incubation periods, the candidate drug is applied to the cultured tissues and/or cells (e.g., hours, days, weeks, or months), and the treated brain tissues/cells are examined using the methods described above.

In some cases, for example, in a method of screening a biomarker, samples (e.g., cells, tissues or other samples containing DNA or RNA, samples containing protein, and/or samples containing metabolite) derived from the non-human animal, part thereof, or offspring thereof according to the present invention may be collected before and after the occurrence of indications associated with a neurological disease or condition (e.g., Aβdeposition, neurofibrillary tangles, morphological or functional (atrophic) synapses, neuronal cell death, or impaired memory and learning function), and then a genetic transcript (transcriptome), genetic translation (proteome), lipid (lipid group), or metabolite (metabolite) from the samples is analyzed comprehensively, and substances that have changed before and after the occurrence of the associated indications are identified. In some embodiments, the samples may include the bodily fluid of the non-human animal, such as blood, plasma (e.g., plasma including exosome), serum, urine, sweat, tears, saliva, semen, and/or cerebrospinal fluid of the rat.

A nucleic acid microarray chip, such as a DNA microarray chip, can be used for analyzing the gene transcription product (e.g., transcriptome); gel electrophoresis (such as two-dimensional gel electrophoresis) or mass spectrometry (such as time-of-flight mass spectrometry, electron spray ionization mass spectrometry, capillary HPLC/MS and LC/MS/MS can be used for analyzing the gene transcription product (e.g., proteome)); NMR, capillary electrophoresis, LC/MS and LC/MS/MS can be used for analyzing the metabolite (metabolome).

When the existence/content of a certain substance shows significant difference before and after the occurrence of related indications, the substance can be regarded as the biomarker for improving the learning ability, treating cognitive impairment and/or treating neurodegenerative disease of the subjects.

Without intending to be limited by any theory, the following embodiments are only intended to illustrate the fusion protein, preparation method and use according to the present application, and are not intended to limit the scope of the present application.

### Embodiments

### Embodiment 1 Overexpression of ATP6V1B2 protein to improve learning and memory functions

### Test steps:

Aged mice of 15 months old were randomly divided into a control group and a test group; adeno-associated viruses (pAAV2/9-hSyn-ATP6V1B2-Myc-2A- mCherry, the amino acid sequence of the ATP6V1B2-mCherry fusion protein is as set forth in SEQ ID NO. 6) carrying ATP6V1B2-mCherry fusion protein or control adeno-associated viruses carrying mCherry were respectively injected into the hippocampus on the back sides of the two sides of the brains of the mice in the test group and the control group, so that the neurons of the hippocampus on the back sides of the two sides of the brains of the mice in the test group and the control group respectively over-expressed the ATP6V1B2-mCherry protein or the mCherry protein. One month after the operation, the mice were subjected to a water maze test. (The test steps are shown in an Embodiment 4).

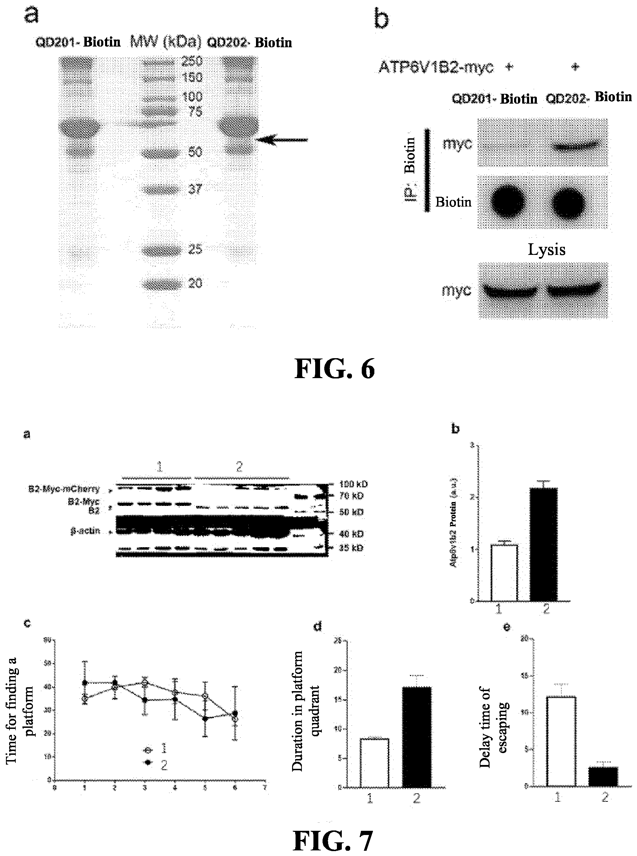

The result shows that the ATP6V1B2 protein (shown in FIGS. 7a, b) is over-expressed in the hippocampus of the aged mice of 15 months old, and the learning and memory abilities of the aged mice in the water maze behavior test can be obviously improved (shown in FIGS. 7c, d, e).

### Embodiment 2 Polypeptide with ATP6V1B2 protein as action target

Test steps (refer to E. Harlow D. Ryan, U.S. E. Harlow D. Ryan, Shen Guanxin, et al. Using Antibodies: A Laboratory Manual [J]. Science Press, 2002):
(1) Polypeptide QD202 was synthesized according to the amino acid sequence as set forth in SEQ ID NO. 1, and a biotin label was added at the amino C' end of the polypeptide; and the amino acid sequence of the QD202 was randomly disorganized, a disordered control QD201 (the amino acid sequence as set forth in SEQ ID NO. 7) was synthesized, and the biotin label was added at the amino C' end of the polypeptide. The polypeptide QD202 and the QD201 were dissolved by PBS, and a storage solution is prepared with the concentration of 500 µM.
(2) The biotin-labeled QD202 and QD201 were respectively feed to 2 C57BL/6 mice of 8 months old through veins at the dosage of 6.25 mg/kg. The mice were anesthetized after 4 h, brain hippocampus tissues were collected, a lysis buffer solution was added, the tissues were crushed by mechanical grinding, and then supernatant was collected after centrifuging. The biotin antibody and Protein G agarose beads were added into the supernatant for immunoprecipitation, the test steps referred to Using Antibodies: A Laboratory Manual [J]. Science Press, 2002. The proteins were eluted by a protein loading buffer solution.
(3) SDS-PAGE protein electrophoresis gel was prepared, the concentration of lower-layer separation gel was 10%, and the concentration of upper-layer concentrated gel was 4%; a sample was added into a loading hole; and SDS-PAGE electrophoresis was carried. After the electrophoresis was ended, the separation gel was cut off, and subjected to staining with Coomassie brilliant blue, with the result shown in FIG. 6a.
(4) A specific band which was displayed by Coomassie brilliant blue staining and had the molecular weight of about 55 kDa was cut off and subjected to mass spectrum pretreatment; enzymolysis was performed on protein to obtain polypeptide fragments; and desalting treatment was carried out. Ion fragments of the sample was divided by mass number through a mass analyzer of the mass spectrum, and a mass spectrum was obtained through a detector; and the sequence of the identified polypeptide fragments was compared in a protein database through search software to identify the protein type.
(5) The disordered control QD201 was compared to detect that the protein specifically bound to QD202 was ATP6V1B2.
(6) A murine Atp6V1b2 expression plasmid with a myc tag at the 3' end was constructed; the plasmid was transfected in HEK 293 cells; the cells were collected after 2 days; and cell protein was extracted. The biotin antibody and protein G agarose beads were added into a cell lysis solution for co-immunoprecipitation^{[1]}, and the protein was eluted by the protein loading buffer solution.
(7) SDS-PAGE protein electrophoresis gel was prepared, the concentration of lower-layer separation gel was 10%, and the concentration of upper-layer concentrated gel was 4%; the sample was added into a loading hole; and the protein was detected by an immunoblotting method, with the result shown in FIG. 6b. The specific binding between the QD202 and the ATP6V1B2 protein can be found by a co-immunoprecipitation method.

The result shows that the binding between the QD202 and the ATP6V1 B2 protein can be found by a protein mass spectrum analysis and co-immunoprecipitation method. The result is shown in FIGS. 6a, b.

### Embodiment 3 Biological activity test of polypeptide

### Test steps:

Polypeptide QD202 was synthesized according to the amino acid sequence as set forth in SEQ ID NO. 1.

Polypeptide QD202 N'-1 was synthesized according to the amino acid sequence as set forth in SEQ ID NO. 2.

Polypeptide QD202 C'-1 was synthesized according to the amino acid sequence as set forth in SEQ ID NO. 3.

Polypeptide QD202 C'-2 was synthesized according to the amino acid sequence as set forth in in SEQ ID NO. 4.

0.1-0.15 ml of 20% ethyl carbamate was injected into the abdominal cavity of a C57 mouse of 2 to 3 months old for anesthesia; a head was quickly cut, and brain tissues were stripped and put into artificial cerebrospinal fluid (ACSF) ice-water mixed liquid into which mixed gas (95%O₂ and 5%CO₂) was introduced in advance, and stood for 2 min; the ACSF included the following components: 11.7 mM of NaCl, 0.36 mM of KCl , 0.12 mM of NaH₂PO₄, 0.25 mM of CaCl₂, 0.12 mM of MgCl₂, 25 mM of NaHCO₃ and 11 mM of glucose. A hippocampus coronal brain slice of 350 µm was cut by a vibration slicer. The hippocampus coronal brain slice was placed into the ACSF at room temperature for more than 30 min for recovery, and then the brain slice was put into a record slot; continuous perfusing was performed at a speed of 2 to 2.5 ml/min by the ACSF into which the mixed gas was introduced; blind-binding sealing was performed on hippocampus CA1 pyramidal neuron under an Olympic BX51 upright microscope; whole-cell patch-clamp was performed by an Axon700B amplifier and a 1550B digital-to-analog converter; spontaneous excitatory postsynaptic current (sEPSC) of the pyramidal neuron was recorded by a voltage clamp under the clamp voltage of -70 mV; after recording for 5 min, synthetic polypeptides such as 5 µM QD202 or QD202 N'-1, C'-1 and C'-2 for 5 min; and then perfusing and flushing were performed for 10 min by the ACSF. The recorded data was analyzed by Minianalysis software.

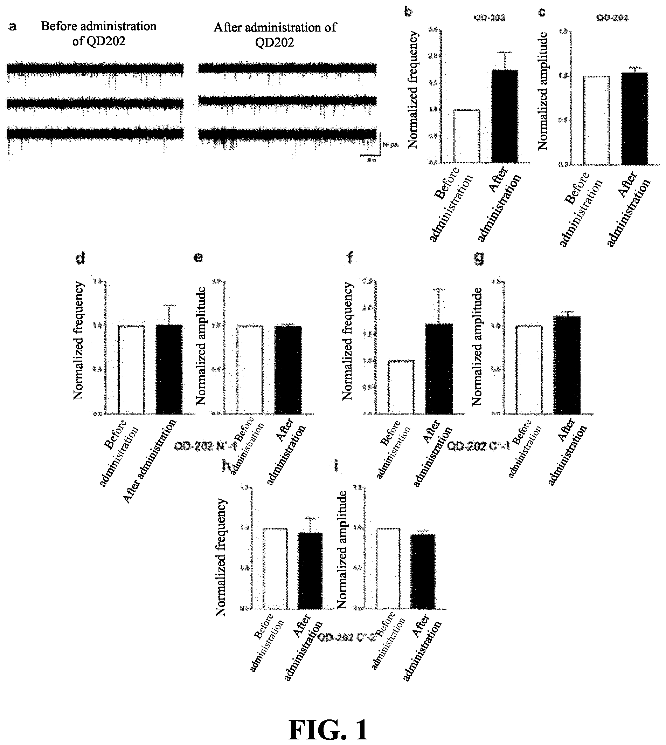

The result shows that QD202 can increase the spontaneous excitatory synaptic transmitter release of hippocampal CA1 neurons, which is mainly reflected in increasing the release frequency of excitatory postsynaptic current, but has no influence on the excitatory postsynaptic current amplitude (see FIGS. 1a, b, c);
compared with the effect of QD202 on excitatory synaptic transmission, the effect similar to that of QD202 cannot be observed by removing one amino acid at the N end (see FIGS. 1d, e); the effect similar to that of QD202 can still be observed by removing one amino acid at the C end (see FIGS. 1f, g); and the effect similar to that of QD202 cannot be observed by removing two amino acids at the C end (see FIGS. 1h, i).

### Embodiment 4 Improvement effect of polypeptides on learning ability

### New object recognition test

A new object recognition test was carried out by an open field box (40×40×35cm, made of blue opaque plastic) with reference to a behavior test scheme recorded by Leger, M., et al. Object recognition test in mice. Nat Protoc. 8, 2531-2537 (2013). On the first day, mice were put into the open field box to adapt for 10 min. On the second day, each mouse was lightly put in the center of the box, two similar objects (a battery I) were put in the central area and freely explore for 10 min, and then the mice were sent back to feeding cages. After 3 h, the mice were put back to the box again (one battery was replaced with a doll toy which was 10 cm high) for 10 min to carry out a memory retention test. A video was analyzed by Etho Vision XT 14 software, and the time for exploring new/old objects by the mice was recorded. The discrimination index was computed as (Tnovel-Tfamliar)/(Tnovel+Tfamiliar).

### Morris water maze test

Morris water maze referred to the test solutions recorded in Qing-Feng Wu et al., Fibroblast growth factor 13 is a microtubule-stabilizing protein modulating neuronal polarization and migration. Cell. 149, 1549-1564 (2012). The test was carried out in a circular pool filled with water (120 cm in diameter, 30 cm in depth, titanium dioxide was added to make opaque, kept temperature at 21±1°C) in a room with a fixed environment. The test was divided into an adaptation period (1 day), a training period (5-6 days) and a testing period (1 day). Adaptation period: the platform was placed 0.5 cm above the water surface, and the mice were guided to the platform. Training day: the platform was placed 0.5 cm underwater, and the mice were trained to find the platform. The timing was stopped when the mice reached the platform, and if the mice did not reach the platform within 1 min, the mice were guided to the platform and allowed to stay on the platform for 30 s. Training were performed 4 times a day, 4 times a day (between 8:00 a.m. to 4:00 p.m.), the mice were dropped from different water points each time, each training was performed at interval of at least 30 min, a total of 6 days, and the order of water points was different every day. Test Day: test was performed 24 h after the end of the training day. The platform was removed, the mice entered the water from untrained water entry points, and videoed for 1 min. The footage was analyzed with Etho Vision XT 14 software, recording parameters including the escape latency of entering the target quadrant, the number of platform crossings, and the dwell time in the target quadrant.

### 4.1 Oral administration of polypeptide

APP/PS1 transgenic mice of 10 months old (purchased from MODEL ORGANISMS) were selected, and equally divided into three groups; the mice were administrated with a PBS buffer solution (group 2),

QD202 (group 3, the administration dosage of QD202 was 6.25 mg/kg (the concentration of QD202 was 500 µM)), and memantine (group 4, the administration dosage of memantine was 1.25 mg/kg (the concentration of memantine was 1 mM)) through intragastric administration; and C57 BL/6 mice of 10 months old without taking any reagent through intragastric administration were taken as a control (group 1).

### Behavioral test was performed on the four groups of mice.

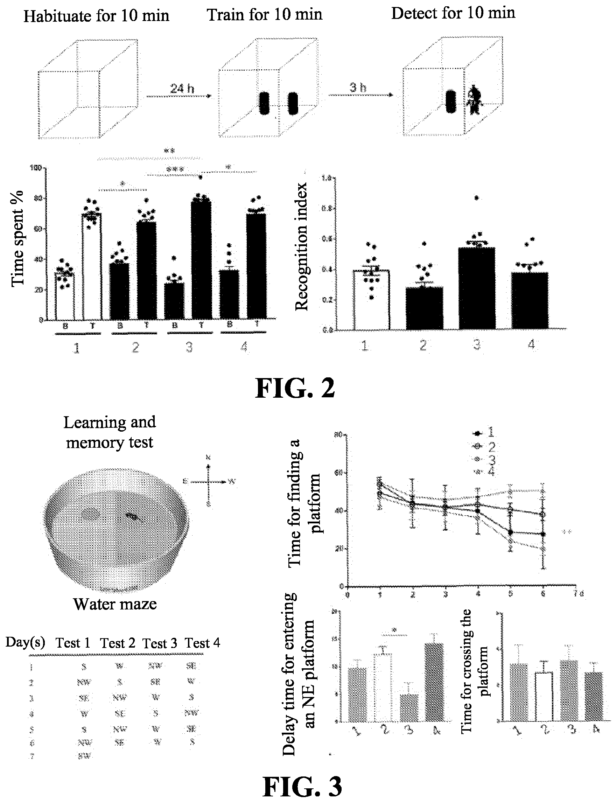

The result shows that after QD202 was continuously administrated in an intragastric manner for 1 week, the APP/PS1 transgenic AD mice show obvious improvement of learning and cognitive functions in new object recognition (shown in FIG. 2) and water maze behavior (shown in FIG. 3) detection.

### 4.2 Intravenous injection of polypeptide

APP/PS1 transgenic mice of 10 months old (purchased from MODEL ORGANISMS) were selected and equally divided into two groups; the mice were administrated with a PBS buffer solution (group 2, concentration of PBS was 500 µM), and QD202 (group 3, the administration dosage of QD202 was 1.25 mg/kg, the concentration of QD202 was 500 µM) through intravenous injection administration; and C57 BL/6 mice of 10 months old without taking any reagent through intravenous injection administration were taken as a control (group 1)..

### Behavioral test was performed on the four groups of mice.

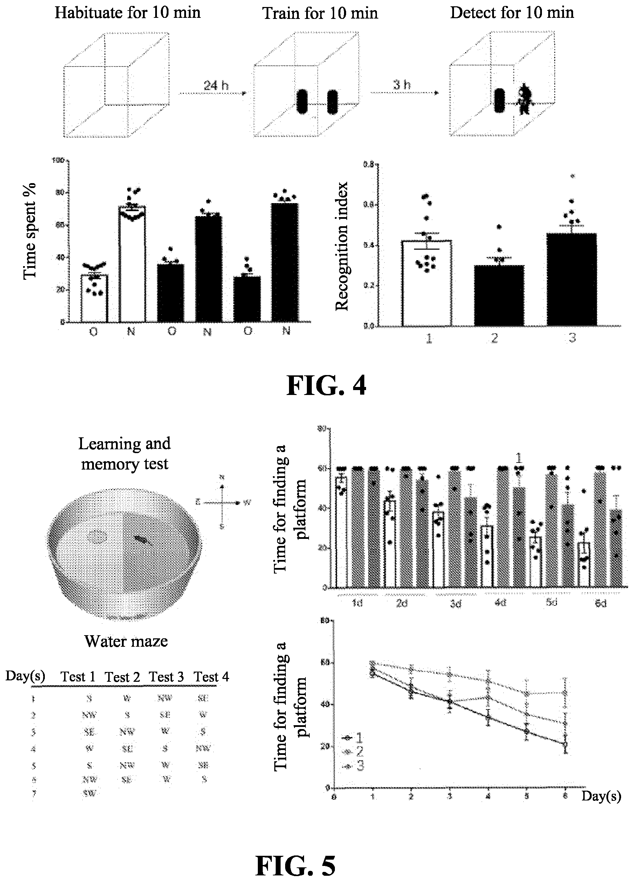

The result shows that after QD202 is continuously injected in an intravenous manner for 1 week, the APP/PS1 transgenic AD mice show obvious improvement on learning and cognitive functions in new object recognition (shown in FIG. 4) and water maze behavior (shown in FIG. 5) detection.

Therefore, QD202 has an obvious effect of improving the learning ability, particularly the memory ability and cognitive ability.

### Embodiment 5 Modified polypeptide, and improvement effect thereof on learning ability

### 5.1 Polypeptide in dimer form

Preparation of a QD202 dimer: QD202 was dissolved with the concentration of 10mg/ml in water, dilute ammonia water was added to regulate the pH value to 8-9, and stirring and oxidizing were performed for 24 h; the reaction endpoint was monitored by HPLC during the period; and purifying was performed by HPLC after complete oxidation to obtain the QD202 dimer. The QD202 dimer included the amino acid sequence as set forth in SEQ ID NO. 18.

According to the method recorded in the Embodiment 4, the QD202 dimer was used for performing cone neuron spontaneous excitatory postsynaptic current detection on the mice. The contrast referred to the spontaneous excitatory postsynaptic current result recorded during normal perfusion of artificial cerebrospinal fluid.

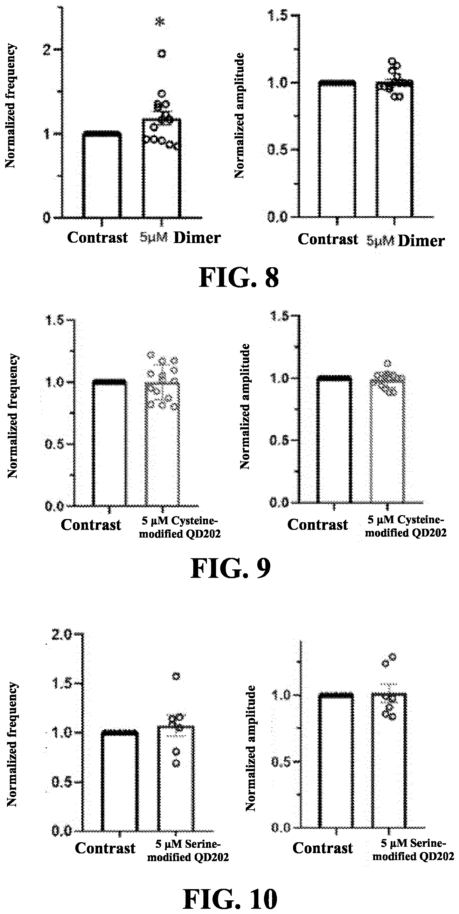

The result is shown in FIG. 8. The result shows that the QD202 dimer can increase spontaneous excitatory synaptic transmitter release of hippocampal CA1 neurons, which is mainly reflected in increasing the release frequency of excitatory postsynaptic current and increasing the excitatory postsynaptic current amplitude.

### 5.2 Cysteine-modified polypeptide

Preparation of cysteine-modified QD202: Fmoc-Cys(CAm)-OH was used for replacing Fmoc-Cys(Trt)-OH, and direct synthesizing was carried out according to the amino acid sequence of QD202, so as to obtain the cysteine-modified QD202.

Cone neuron spontaneous excitatory postsynaptic current detection was performed on the mice by the cysteine-modified QD202 according to the method recorded in the Embodiment 4. The contrast referred to the spontaneous excitatory postsynaptic current result recorded during normal perfusion of artificial cerebrospinal fluid.

The result is shown in FIG. 9. The result shows that the cysteine-modified QD202 increases the disappearance of the activity of the spontaneous excitatory synaptic transmitter release of hippocampal CA1 neurons, which is mainly reflected in reducing the release frequency of excitatory postsynaptic current and/or reducing the excitatory postsynaptic current amplitude.

### 5.3 Serine-modified polypeptide

Preparation of serine-modified polypeptide QD202: Fmoc-Ser(HPO3Bzl)-OH was used for replacing Fmoc-Ser(tbu)-OH, and direct synthesizing was carried out according to the amino acid sequence of QD202, so as to obtain the serine-modified polypeptide QD202 subjected to phosphorylation modification at 12th serine (S) from the N end.

Cone neuron spontaneous excitatory postsynaptic current detection was performed on the mice by the serine-modified QD202 according to the method recorded in the Embodiment 4. The contrast referred to the spontaneous excitatory postsynaptic current result recorded during normal perfusion of artificial cerebrospinal fluid.

The result is shown in FIG. 10. The result shows that the serine-modified QD202 increases the disappearance of the activity of the spontaneous excitatory synaptic transmitter release of hippocampal CA1 neurons, which is mainly reflected in reducing the release frequency of excitatory postsynaptic current and/or reducing the excitatory postsynaptic current amplitude.

### Embodiment 6 Binding site of polypeptide to ATP6V1B2 subunit

Expression plasmids including murine ATP6V1B2 full-length sequence, 1-286aa truncated sequence or 1-172aa truncated sequence with a Myc tag are respectively constructed; the amino acid sequence of the murine ATP6V1B2 full-length sequence was as set forth in SEQ ID NO. 8, the amino acid sequence of the murine ATP6V1B21-286aa was as set forth in SEQ ID NO. 10; the amino acid sequence of the murine ATP6V1B21-172aa as set forth in as SEQ ID NO. 11; and these plasmids were respectively called as an ATP6V1B2 full-length expression plasmid, an ATP6V1B21-286aa expression plasmid and an ATP6V1B21-172aa expression plasmid. Meanwhile, a QD202 expression plasmid with an EGFP tag was constructed.

The QD202 expression plasmid, the ATP6V1B2 full-length expression plasmid, the ATP6V1B21-286aa expression plasmid or the ATP6V1B21-172aa expression plasmid were simultaneously transfected in 293 cells (purchased from Cell Bank/Stem Cell Bank of CHINESE ACADEMY OF SCIENCES), a cell lysis solution was collected after 48, and a Myc antibody (purchased from Sigma-Aldrich) was added into the lysis solution to enrich ATP6V1B2 full-length protein, 1-286aa truncated sequence or 1-172aa truncated sequence.

Then a Protein G agarose bead was added to be bound to the Myc antibody, so as to precipitate the ATP6V1B2 full-length protein, the 1-286aa truncated sequence or the 1-172aa truncated sequence, and proteins which were bound to one another, and finally the proteins subjected to co-immunoprecipitation was detected by virtue of an immunoblotting method.

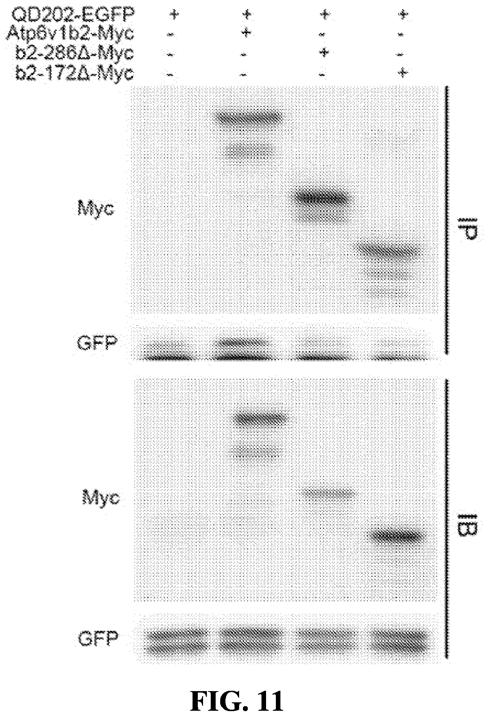

The result is shown in FIG. 11, Atp6vlb2-Myc, b2-286Δ-Myc and b2-172Δ-Myc respectively correspond to the ATP6V1B2 full-length protein, the 1-286aa truncated sequence or the 1-172aa truncated sequence. The result shows that only the full-length ATP6V1B2 protein can be combined with a QD202 polypeptide, so that the binding site of ATP6V1B2 and QD202 is positioned in a region range from 288th amino acid to 512th amino acid at the N-terminal of ATP6V1B2.

The foregoing details are provided by way of explanation and illustration and are not intended to limit the scope of the attached claims. Various modifications to the embodiments described herein will be apparent to those of ordinary skill in the art and remain within the scope of the appended claims and their equivalents.

## Claims

1. A use of a proton pump modulator in preparing a reagent, wherein the reagent is used for improving learning ability.

2. A use of a proton pump modulator in preparing a reagent, wherein the reagent is used for treating cognitive impairment.

3. A use of a proton pump modulator in preparing a reagent, wherein the reagent is used for treating neurodegenerative diseases.

4. The use according to any one of claims 1 to 3, wherein the reagent improves the expression level and/or activity of proton pump-associated protein in a subject.

5. The use according to any one of claims 1 to 4, wherein the proton pump modulator improves the expression level and/or activity of the proton pump-associated protein in the subject.

6. The use according to any one of claims 4 to 5, wherein the expression level of the proton pump-associated protein comprises the expression level of a gene encoding the proton pump-associated protein, the transcription level of the gene encoding the proton pump-associated protein, and/or the expression level of the proton pump-associated protein.

7. The use according to any one of claims 4 to 6, wherein the improvement comprises that the expression level and/or activity of the proton pump-associated protein is improved by at least about 10% as compared with the expression level and/or activity of original proton pump-associated protein in the subject.

8. The use according to any one of claims 4 to 7, wherein the expression level of the proton pump-associated protein is measured by implementing a test selected from the following groups: qPCR, qRT-PCR, hybridization analysis, RNA Northern blotting, dot blotting, in-situ hybridization, gel electrophoresis, capillary electrophoresis, column chromatography, western blotting, immunohistochemistry, immunostaining and mass spectrometry.

9. The use according to any one of claims 4 to 8, wherein the expression level of the proton pump-associated protein is measured by a substance selected from the following groups: a primer capable of specifically amplifying the gene encoding the proton pump-associated protein, a nucleic acid molecule specifically bound to the gene encoding the proton pump-associated protein, a nucleic acid molecule specifically bound to the proton pump-associated protein, a small molecule specifically bound to the proton pump-associated protein, a probe specifically bound to the proton pump-associated protein, and polypeptide specifically bound to the proton pump-associated protein.

10. The use according to any one of claims 1 to 9, wherein the reagent improves the expression level and/or activity of ATP6V1B2 in the subject.

11. The use according to any one of claims 1 to 10, wherein the proton pump modulator improves the expression level and/or activity of ATP6V1B2 in the subject.

12. The use according to any one of claims 10 to 11, wherein the expression level of the A TP6V 1 B2 comprises the expression level of an ATP6V1B2 gene, the transcription level of the ATP6V1B2 gene, and/or the expression level of ATP6V1B2 protein.

13. The use according to any one of claims 10 to 12, wherein the improvement comprises that the expression level and/or activity of ATP6V1B2 is improved by at least about 10% as compared with the expression level and/or activity of original ATP6V1B2 in the subject.

14. The use according to any one of claims 10 to 13, wherein the expression level of the ATP6V1B2 is measured by implementing a test selected from the following groups: qPCR, qRT-PCR, hybridization analysis, RNA Northern blotting, dot blotting, in-situ hybridization, gel electrophoresis, capillary electrophoresis, column chromatography, western blotting, immunohistochemistry, immunostaining and mass spectrometry.

15. The use according to any one of claims 10 to 14, wherein the expression level of ATP6V1B2 is measured by a substance selected from the following groups: a primer capable of specifically amplifying the ATP6V1B2 gene, a nucleic acid molecule specifically bound to the ATP6V1B2 gene, a nucleic acid molecule specifically bound to the ATP6V1B2 protein, a small molecule specifically bound to the ATP6V1B2 protein, a probe specifically bound to the ATP6V1B2 protein, and polypeptide specifically bound to the ATP6V1B2 protein.

16. The use according to any one of claims 1 to 15, wherein the proton pump modulator comprises a proton pump agonist.

17. The use according to any one of claims 1 to 16, wherein the proton pump modulator comprises a vesicle-type proton pump agonist.

18. The use according to any one of claims 1 to 17, wherein the proton pump modulator comprises an agonist of the ATP6V1B2 protein.

19. The use according to any one of claims 1 to 18, wherein the proton pump modulator is capable of binding to the ATP6V1B2 protein.

20. The use according to any one of claims 1 to 19, wherein the proton pump modulator is capable of binding to at least part of sequence of the 288th to 512th amino acid sequence of mouse ATP6V1B2 protein.

21. The use according to any one of claims 1 to 20, wherein the proton pump modulator is capable of binding to at least part of sequence of the 288th to 512th amino acid sequence of human ATP6V1B2 protein.

22. The use according to any one of claims 1 to 21, wherein the proton pump modulator comprises nucleic acid or a variant thereof, polypeptide or a variant thereof, and/or small molecule

23. The use according to any one of claims 1 to 22, wherein the proton pump modulator is capable of increasing the synaptic neurotransmitter release.

24. The use according to claim 23, wherein the increase comprises that at least about 10% is increased as compared with the original synaptic neurotransmitter release level in the subject.

25. The use according to any one of claims 1 to 24, wherein the proton pump modulator is capable of increase the release frequency of excitatory postsynaptic current.

26. The use according to claim 25, wherein the increase comprises that at least about 10% is increased as compared with the release frequency level of the excitatory postsynaptic current in the subject.

27. The use according to any one of claims 1, 4 to 26, wherein the learning ability comprises cognitive ability, motor ability, memory ability, and/or spatial exploration ability.

28. The use according to any one of claims 1, 4 to 27, wherein the improvement of the learning ability comprises that compared with an evaluation score of original learning ability of the subject, the evaluation score of the improved learning ability of the subject is increased by at least about 50%.

29. The use according to any one of claims 1, 4 to 28, wherein the evaluation score of the learning ability is measured by implementing a test selected from the following groups: a new object cognitive test and a water maze test.

30. The use according to claim 29, wherein the new object cognitive test is used for evaluating the cognitive ability, motor ability and/or spatial exploration ability.

31. The use according to any one of claims 29 to 30, wherein the water maze test is used for evaluating the memory ability, motor ability and/or spatial exploration ability.

32. The use according to any one of claims 4 to 31, wherein the subject comprises mammals.

33. The use according to any one of claims 4 to 32, wherein the subject comprises human.

34. The use according to any one of claims 4 to 33, wherein the subject comprises a patient with non-cognitive impairment and/or a patient with non-neurodegenerative disease.

35. The use according to any one of claims 4 to 34, wherein the subject comprises a patient with neurodegenerative disease and/or a patient with cognitive impairment.

36. The use according to any one of claims 4 to 35, wherein the subject comprises a patient with Alzheimer disease.

37. The use according to any one of claims 4 to 36, wherein the subject is in an aged stage.

38. The use according to any one of claims 2, 4 to 37, wherein the cognitive impairment comprises mild cognitive impairment (MCI), middle cognitive impairment and late cognitive impairment.

39. The use according to any one of claims 2, 4 to 38, wherein the cognitive impairment comprises cognitive impairment caused by normal aging, Lews Body Dementia (LBD), frontotemporal dementia and/or vascular dementia.

40. The use according to any one of claims 2, 4 to 39, wherein the diseases induced by the cognitive impairment comprises Alzheimer disease, multi-infarct, Parkinson's disease, AIDS and/or Creutzfeldt-Jakob disease (CJD).

41. The use according to any one of claims 3 to 40, wherein the neurodegenerative disease comprises acute neurodegenerative disease and chronic neurodegenerative disease.

42. The use according to any one of claims 3 to 41, wherein the neurodegenerative disease comprises neurodegenerative disease caused by neuronal death and glial cell homeostasis, neurodegenerative disease caused by aging, neurodegenerative disease caused by influence on the CNS cell function, neurodegenerative disease caused by abnormal communication between cells, and/or neurodegenerative disease caused by damage to cell motor.

43. The use according to any one of claims 3 to 42, wherein the neurodegenerative disease comprises Alzheimer disease, Parkinson's disease, multiple sclerosis (MS), amyotrophic lateral sclerosis (ALS), and/or Huntington's disease (HD).

44. The use according to any one of claims 1 to 43, wherein the reagent is formulated to be suitable for oral administration and/or injection administration.

45. The use according to any one of claims 1 to 44, wherein the reagent is formulated to be suitable for intravenous injection.

46. A use of ATP6V1B2, a functional active fragment thereof, or a nucleic acid molecule encoding same in improvement of learning ability.

47. A use of ATP6V1B2, a functional active fragment thereof, or a nucleic acid molecule encoding same in treatment of cognitive impairment.

48. A use of ATP6V1B2, a functional active fragment thereof, or a nucleic acid molecule encoding same in treatment of neurodegenerative diseases.

49. The use according to any one of claims 46 to 48, wherein the ATP6V1B2 is derived from human.

50. The use according to any one of claims 46 to 49, wherein the ATP6V1B2 comprises the amino acid sequence as set forth in SEQ ID NO. 8 or 16.

51. The use according to any one of claims 46 to 50, wherein the functional active fragment of the ATP6V1B2 has the capacity of specifically binding to the amino acid sequence as set forth in SEQ ID NO. 5.

52. The use according to any one of claims 46 to 51, wherein the functional active fragment of the ATP6V1B2 comprises a truncation of the ATP6V1B2.

53. The use according to any one of claims 46 to 52, wherein the functional active fragment of the ATP6V1B2 comprises at least part of sequence of the 288th-512th amino acid sequence of human ATP6V1B2 protein.

54. The use according to any one of claims 46 to 53, wherein the functional active fragment of the ATP6V1B2 comprises at least part of sequence of the 288th-512th amino acid sequence of mouse ATP6V1B2 protein.

55. The use according to any one of claims 46 to 54, wherein the functional active fragment of the ATP6V1B2 comprise the amino acid sequence as set forth in any one of SEQ ID NO. 10-11.

56. The use according to any one of claims 46 to 55, wherein the nucleic acid molecule comprises the nucleotide sequence as set forth in SEQ ID NO. 9 or 17.

57. The use according to any one of claims 46, 49 to 56, wherein the learning ability comprises cognitive ability, motor ability, memory ability, and/or spatial exploration ability.

58. The use according to any one of claims 46, 49 to 57, wherein the improvement of the learning ability comprises that compared with an evaluation score of original learning ability of the subject, the evaluation score of the improved learning ability of the subject is increased by at least about 50%.

59. The use according to any one of claims 46, 49 to 58, wherein the evaluation score of the learning ability is measured by implementing a test selected from the following groups: a new object cognitive test and a water maze test.

60. The use according to claim 59, wherein the new object cognitive test is used for evaluating the cognitive ability, motor ability and/or spatial exploration ability.

61. The use according to any one of claims 59 to 60, wherein the water maze test is used for evaluating the memory ability, motor ability and/or spatial exploration ability.

62. The use according to any one of claims 58 to 61, wherein the subject comprises mammals.

63. The use according to any one of claims 58 to 62, wherein the subject comprises human.

64. The use according to any one of claims 58 to 63, wherein the subject comprises a patient with non-cognitive impairment and/or a patient with non-neurodegenerative disease.

65. The use according to any one of claims 58 to 64, wherein the subject comprises a patient with neurodegenerative disease and/or a patient with cognitive impairment.

66. The use according to any one of claims 58 to 65, wherein the subject comprises a patient with Alzheimer disease.

67. The use according to any one of claims 58 to 66, wherein the subject is in an aged stage.

68. The use according to any one of claims 47, 49 to 67, wherein the cognitive impairment comprises mild cognitive impairment (MCI), middle cognitive impairment and late cognitive impairment.

69. The use according to any one of claims 47, 49 to 68, wherein the cognitive impairment comprises cognitive impairment caused by normal aging, Lews Body Dementia (LBD), frontotemporal dementia and/or vascular dementia.

70. The use according to any one of claims 47, 49 to 69, wherein the diseases induced by the cognitive impairment comprises Alzheimer disease, multi-infarct, Parkinson's disease, AIDS and/or Creutzfeldt-Jakob disease (CJD).

71. The use according to any one of claims 48 to 70, wherein the neurodegenerative disease comprises acute neurodegenerative disease and chronic neurodegenerative disease.

72. The use according to any one of claims 48 to 71, wherein the neurodegenerative disease comprises neurodegenerative disease caused by neuronal death and glial cell homeostasis, neurodegenerative disease caused by aging, neurodegenerative disease caused by influence on the CNS cell function, neurodegenerative disease caused by abnormal communication between cells, and/or neurodegenerative disease caused by damage to cell motor.

73. The use according to any one of claims 48 to 72, wherein the neurodegenerative disease comprises Alzheimer disease, Parkinson's disease, multiple sclerosis (MS), amyotrophic lateral sclerosis (ALS), and/or Huntington's disease (HD).

74. A use of an ATP6V1B2 modulator in preparing a reagent, wherein the reagent is used for improving learning ability.

75. A use of an ATP6V1B2 modulator in preparing a reagent, wherein the reagent is used for treating cognitive impairment.

76. A use of an ATP6V1B2 modulator in preparing a reagent, wherein the reagent is used for treating neurodegenerative diseases.

77. The use according to any one of claims 74 to 76, wherein the reagent improves the expression level and/or activity of ATP6V1B2 in the subject.

78. The use according to any one of claims 74 to 77, wherein the ATP6V1B2 modulator improves the expression level and/or activity of ATP6V1B2 in the subject.

79. The use according to any one of claims 74 to 78, wherein the expression level comprises the expression level of an ATP6V1B2 gene, the transcription level of the ATP6V1B2 gene and/or the expression level of ATP6V1B2 protein.

80. The use according to any one of claims 77 to 79, wherein the activity improvement comprises the improvement of the expression level and/or activity of proton pump-associated protein.

81. The use according to any one of claims 77 to 80, wherein the improvement comprises that the expression level and/or activity of ATP6V1B2 is improved by at least about 10% as compared with the expression level and/or activity of original ATP6V1B2 in the subject.

82. The use according to any one of claims 77 to 81, wherein the expression level of the ATP6V1B2 is measured by implementing a test selected from the following groups: qPCR, qRT-PCR, hybridization analysis, RNA Northern blotting, dot blotting, in-situ hybridization, gel electrophoresis, capillary electrophoresis, column chromatography, western blotting, immunohistochemistry, immunostaining and mass spectrometry.

83. The use according to any one of claims 77 to 82, wherein the expression level of ATP6V1B2 is measured by a substance selected from the following groups: a primer capable of specifically amplifying the ATP6V1B2 gene, a nucleic acid molecule specifically bound to the ATP6V1B2 gene, a nucleic acid molecule specifically bound to the ATP6V1B2 protein, a small molecule specifically bound to the ATP6V1B2 protein, a probe specifically bound to the ATP6V1B2 protein, and polypeptide specifically bound to the ATP6V1B2 protein.

84. The use according to any one of claims 74 to 83, wherein the ATP6V1B2 modulator comprises an agonist of the ATP6V1B2 protein.

85. The use according to any one of claims 74 to 84, wherein the ATP6V1B2 modulator is capable of binding to the ATP6V1B2 protein.

86. The use according to any one of claims 74 to 85, wherein the ATP6V1B2 modulator is capable of binding to at least part of sequence of the 288th to 512th amino acid sequence of mouse ATP6V1B2 protein.

87. The use according to any one of claims 74 to 86, wherein the ATP6V1B2 modulator is capable of binding to at least part of sequence of the 288th to 512th amino acid sequence of human ATP6V1B2 protein.

88. The use according to any one of claims 74 to 87, wherein the ATP6V1B2 modulator comprises a proton pump agonist.

89. The use according to any one of claims 74 to 88, wherein the ATP6V1B2 modulator comprises a vesicle-type proton pump agonist.

90. The use according to any one of claims 74 to 89, wherein the ATP6V1B2 modulator comprises nucleic acid or a variant thereof, polypeptide or a variant thereof, and/or small molecule

91. The use according to any one of claims 74 to 90, wherein the ATP6V1B2 modulator comprises polypeptide or the variant thereof.

92. The use according to claim 91, wherein the polypeptide or variant thereof is capable of specifically binding to the ATP6V1B2 protein.

93. The use according to any one of claims 91 to 92, wherein the polypeptide or variant thereof is capable of increasing the synaptic neurotransmitter release.

94. The use according to claim 93, wherein the increase comprises that at least about 10% is increased as compared with the original synaptic neurotransmitter release level in the subject.

95. The use according to any one of claims 91 to 94, wherein the polypeptide or variant thereof is capable of increasing the release frequency of excitatory postsynaptic current.

96. The use according to claim 95, wherein the increase comprises that at least about 10% is increased as compared with the release frequency level of the excitatory postsynaptic current in the subject.

97. The use according to any one of claims 91 to 96, wherein the polypeptide or variant thereof comprises the amino acid sequence as set forth in SEQ ID NO. 5.

98. The use according to any one of claims 91 to 97, wherein the polypeptide or variant thereof comprises the amino acid sequence as set forth in any one of SEQ ID NO. 1 and 3.

99. The use according to any one of claims 74, 77 to 98, wherein the learning ability comprises cognitive ability, motor ability, memory ability, and/or spatial exploration ability.

100. The use according to any one of claims 74, 77 to 99, wherein the improvement of the learning ability comprises that compared with an evaluation score of original learning ability of the subject, the evaluation score of the improved learning ability of the subject is increased by at least about 50%.

101. The use according to any one of claims 74, 77 to 100, wherein the evaluation score of the learning ability is measured by implementing a test selected from the following groups: a new object cognitive test and a water maze test.

102. The use according to claim 101, wherein the new object cognitive test is used for evaluating the cognitive ability, motor ability and/or spatial exploration ability.

103. The use according to any one of claims 101 to 102, wherein the water maze test is used for evaluating the memory ability, motor ability and/or spatial exploration ability.

104. The use according to any one of claims 77 to 103, wherein the subject comprises mammals.

105. The use according to any one of claims 77 to 104, wherein the subject comprises human.

106. The use according to any one of claims 77 to 105, wherein the subject comprises a patient with non-cognitive impairment and/or a patient with non-neurodegenerative disease.

107. The use according to any one of claims 77 to 106, wherein the subject comprises a patient with neurodegenerative disease and/or a patient with cognitive impairment.

108. The use according to any one of claims 77 to 107, wherein the subject comprises a patient with Alzheimer disease.

109. The use according to any one of claims 77 to 108, wherein the subject is in an aged stage.

110. The use according to any one of claims 75, 77 to 109, wherein the cognitive impairment comprises mild cognitive impairment (MCI), middle cognitive impairment and late cognitive impairment.

111. The use according to any one of claims 75, 77 to 110, wherein the cognitive impairment comprises cognitive impairment caused by normal aging, Lews Body Dementia (LBD), frontotemporal dementia and/or vascular dementia.

112. The use according to any one of claims 75, 77 to 111, wherein the diseases induced by the cognitive impairment comprises Alzheimer disease, multi-infarct, Parkinson's disease, AIDS and/or Creutzfeldt-Jakob disease (CJD).

113. The use according to any one of claims 76 to 112, wherein the neurodegenerative disease comprises acute neurodegenerative disease and chronic neurodegenerative disease.

114. The use according to any one of claims 76 to 113, wherein the neurodegenerative disease comprises neurodegenerative disease caused by neuronal death and glial cell homeostasis, neurodegenerative disease caused by aging, neurodegenerative disease caused by influence on the CNS cell function, neurodegenerative disease caused by abnormal communication between cells, and/or neurodegenerative disease caused by damage to cell motor.

115. The use according to any one of claims 76 to 114, wherein the neurodegenerative disease comprises Alzheimer disease, Parkinson's disease, multiple sclerosis (MS), amyotrophic lateral sclerosis (ALS), and/or Huntington's disease (HD).

116. The use according to any one of claims 74 to 115, wherein the reagent is formulated to be suitable for oral administration and/or injection administration.

117. The use according to any one of claims 74 to 116, wherein the reagent is formulated to be suitable for intravenous injection.

118. A method for screening drugs capable of improving learning ability, treating cognitive impairment, and/or treating neurodegenerative diseases of a subject, comprising the following step: detecting the influence of the candidate drugs on the expression level and/or activity of proton pump-associated protein in the subject, wherein after the candidate drugs are applied, the expression level and/or activity of the proton pump-associated protein are improved, and the candidate drugs is capable of improving the learning ability, treat cognitive impairment, and/or treat neurodegenerative diseases of the subject.

119. A method for screening drugs capable of improving learning ability, treating cognitive impairment, and/or treating neurodegenerative diseases of a subject, comprising the following step: detecting the influence of the candidate drugs on the expression level and/or activity of ATP6V1B2 in the subject, wherein after the candidate drugs are applied, the expression level and/or activity of the ATP6V1B2 are improved, and the candidate drugs is capable of improving the learning ability, treat cognitive impairment, and/or treat neurodegenerative diseases of the subject.

120. The method according to claim 118, wherein the expression level of the proton pump-associated protein comprises the expression level of a gene encoding the proton pump-associated protein, the transcription level of the gene encoding the proton pump-associated protein, and/or the expression level of the proton pump-associated protein.

121. The method according to any one of claims 118 and 120, wherein the improvement comprises that the expression level and/or activity of the proton pump-associated protein is improved by at least about 10% as compared with the expression level and/or activity of original proton pump-associated protein in the subject.

122. The method according to any one of claims 118, 120 to 121, wherein the expression level of the proton pump-associated protein is measured by implementing a test selected from the following groups: qPCR, qRT-PCR, hybridization analysis, RNA Northern blotting, dot blotting, in-situ hybridization, gel electrophoresis, capillary electrophoresis, column chromatography, western blotting, immunohistochemistry, immunostaining and mass spectrometry.

123. The method according to any one of claims 118, 120 to 121, wherein the expression level of the proton pump-associated protein is measured by a substance selected from the following groups: a primer capable of specifically amplifying the gene encoding the proton pump-associated protein, a nucleic acid molecule specifically bound to the gene encoding the proton pump-associated protein, a nucleic acid molecule specifically bound to the proton pump-associated protein, a small molecule specifically bound to the proton pump-associated protein, a probe specifically bound to the proton pump-associated protein, and polypeptide specifically bound to the proton pump-associated protein.

124. The method according to any one of claims 119 to 123, wherein the expression level of the ATP6V1B2 comprises the expression level of an ATP6V1B2 gene, the transcription level of the ATP6V1B2 gene and/or the expression level of ATP6V1B2 protein.

125. The method according to any one of claims 119 to 124, wherein the improvement comprises that the expression level and/or activity of ATP6V1B2 is improved by at least about 10% as compared with the expression level and/or activity of original ATP6V1B2 in the subject.

126. The method according to any one of claims 119 to 125, wherein the expression level of the ATP6V1B2 is measured by implementing a test selected from the following groups: qPCR, qRT-PCR, hybridization analysis, RNA Northern blotting, dot blotting, in-situ hybridization, gel electrophoresis, capillary electrophoresis, column chromatography, western blotting, immunohistochemistry, immunostaining and mass spectrometry.

127. The method according to any one of claims 119 to 126, wherein the expression level of ATP6V1B2 is measured by a substance selected from the following groups: a primer capable of specifically amplifying the ATP6V1B2 gene, a nucleic acid molecule specifically bound to the ATP6V1B2 gene, a nucleic acid molecule specifically bound to the ATP6V1B2 protein, a small molecule specifically bound to the ATP6V1B2 protein, a probe specifically bound to the ATP6V1B2 protein, and polypeptide specifically bound to the ATP6V1B2 protein.

128. The method according to any one of claims 118 to 127, wherein the learning ability comprises cognitive ability, motor ability, memory ability, and/or spatial exploration ability.

129. The method according to any one of claims 118 to 128, wherein the administration comprises oral and/or injection administration.

130. The method according to any one of claims 118 to 129, wherein the administration comprises intravenous injection.

131. Polypeptide or variant thereof capable of modulating expression level and/or activity of ATP6V1B2.

132. The polypeptide or variant thereof according to claim 131, wherein the polypeptide or variant thereof is capable of increasing the synaptic neurotransmitter release.

133. The polypeptide or variant thereof according to claim 132, wherein the increase comprises that at least about 10% is increased as compared with the original synaptic neurotransmitter release level in the subject.

134. The polypeptide or variant thereof according to any one of claims 131 to 133, wherein the polypeptide or variant thereof is capable of increasing the release frequency of excitatory postsynaptic current.

135. The polypeptide or variant thereof according to claim 134, wherein the increase comprises that at least about 10% is increased as compared with the release frequency level of the excitatory postsynaptic current in the subject.

136. The polypeptide or variant thereof according to any one of claims 131 to 135, wherein the polypeptide or variant thereof is capable of binding to the ATP6V1B2 protein.

137. The polypeptide or variant thereof according to any one of claims 131 to 136, wherein the polypeptide or variant thereof is capable of improving the expression level and/or activity of the ATP6V1B2 in the subject.

138. The polypeptide or variant thereof according to any one of claim 137, wherein the expression level of the ATP6V1B2 comprises the expression level of an ATP6V1B2 gene, the transcription level of the ATP6V1B2 gene and/or the expression level of ATP6V1B2 protein.

139. The polypeptide or variant thereof according to any one of claims 137 to 138, wherein the improvement comprises that the expression level and/or activity of ATP6V1B2 is improved by at least about 10% as compared with the expression level and/or activity of original ATP6V1B2 in the subject.

140. The polypeptide or variant thereof according to any one of claims 131 to 139, wherein the polypeptide or variant thereof is capable of binding to at least part of sequence of the 288th to 512th amino acid sequence of mouse ATP6V1B2 protein.

141. The polypeptide or variant thereof according to any one of claims 131 to 140, wherein the polypeptide or variant thereof is capable of binding to at least part of sequence of the 288th to 512th amino acid sequence of human ATP6V1B2 protein.

142. The polypeptide or variant thereof according to any one of claims 131 to 141, wherein the polypeptide or variant thereof comprises the amino acid sequence as set forth in SEQ ID NO. 5.

143. The polypeptide or variant thereof according to any one of claims 131 to 142, wherein the polypeptide or variant thereof comprises the amino acid sequence as set forth in any one of SEQ ID NO. 1 and 3.

144. The polypeptide or variant thereof according to any one of claims 131 to 143, wherein the polypeptide or variant thereof comprises a multimer.

145. The polypeptide or variant thereof according to claim 144, wherein the polymer comprises a homodimer.

146. The polypeptide or variant thereof according to any one of claims 131 to 145, wherein cysteine in the amino acid sequence does not have a sulfhydryl blocking modification.

147. The polypeptide or variant thereof according to any one of claims 131 to 146, wherein serine in the amino acid sequence does not have a phosphorylation modification.

148. A pharmaceutical combination, comprising the polypeptide or variant thereof according to any one of claims 131 to 147.

149. A pharmaceutical composition, comprising the polypeptide or variant thereof according to any one of claims 131 to 147, and a pharmaceutically acceptable carrier.

150. A use of the polypeptide or variant thereof according to any one of claims 131 to 147, the pharmaceutical combination according to claim 148, and/or the pharmaceutical composition according to claim 149 in preparing a reagent, wherein the reagent is used for improving learning ability, treating cognitive impairment, and/or treating neurodegenerative diseases.

151. The use according to claim 150, wherein the learning ability comprises cognitive ability, motor ability, memory ability, and/or spatial exploration ability.

152. The use according to any one of claims 150 to 151, wherein the cognitive impairment comprises mild cognitive impairment (MCI), middle cognitive impairment and late cognitive impairment.

153. The use according to any one of claims 150 to 152, wherein the cognitive impairment comprises cognitive impairment caused by normal aging, Lews Body Dementia (LBD), frontotemporal dementia and/or vascular dementia.

154. The use according to any one of claims 150 to 153, wherein the diseases induced by the cognitive impairment comprises Alzheimer disease, multi-infarct, Parkinson's disease, AIDS and/or Creutzfeldt-Jakob disease (CJD).

155. The use according to any one of claims 150 to 154, wherein the neurodegenerative disease comprises acute neurodegenerative disease and chronic neurodegenerative disease.

156. The use according to any one of claims 150 to 155, wherein the neurodegenerative disease comprises neurodegenerative disease caused by neuronal death and glial cell homeostasis, neurodegenerative disease caused by aging, neurodegenerative disease caused by influence on the CNS cell function, neurodegenerative disease caused by abnormal communication between cells, and/or neurodegenerative disease caused by damage to cell motor.

157. The use according to any one of claims 150 to 156, wherein the neurodegenerative disease comprises Alzheimer disease, Parkinson's disease, multiple sclerosis (MS), amyotrophic lateral sclerosis (ALS), and/or Huntington's disease (HD).

158. The use according to any one of claims 150 to 157, wherein the polypeptide, the pharmaceutical combination, and/or the pharmaceutical composition are formulated to be suitable for oral administration and/or injection administration.

159. The use according to any one of claims 150 to 158, wherein the polypeptide, the pharmaceutical combination, and/or the pharmaceutical composition are formulated to be suitable for intravenous injection.

160. A non-human animal or part thereof, comprising and/or expressing ATP6V1B2, a functional active fragment thereof, and/or a nucleic acid molecule encoding same.

161. The non-human animal or part thereof according to claim 160, wherein the ATP6V1B2 is derived from human.

162. The non-human animal or part thereof according to any one of claims 160 to 161, wherein the ATP6V1B2 comprises the amino acid sequence as set forth in SEQ ID NO. 8 or 16.

163. The non-human animal or part thereof according to any one of claims 160 to 162, wherein the functional active fragments of the ATP6V1B2 have the capacity of specifically binding to the amino acid sequence as set forth in SEQ ID NO. 5.

164. The non-human animal or part thereof according to any one of claims 160 to 163, wherein the functional active fragments of the ATP6V1B2 comprise a truncation of the ATP6V1B2.

165. The non-human animal or part thereof according to any one of claims 160 to 164, wherein the functional active fragments of the ATP6V1B2 comprise at least part of sequence of the 288th-512th amino acid sequence of human ATP6V1B2 protein.

166. The non-human animal or part thereof according to any one of claims 160 to 165, wherein the functional active fragments of the ATP6V1B2 comprise at least part of sequence of the 288th-512th amino acid sequence of mouse ATP6V1B2 protein.

167. The non-human animal or part thereof according to any one of claims 160 to 166, wherein the functional active fragments of the ATP6V1B2 comprise the amino acid sequence as set forth in any one of SEQ ID NO. 10-11.

168. The non-human animal or part thereof according to any one of claims 160 to 167, wherein the nucleic acid molecule comprises the nucleotide sequence as set forth in SEQ ID NO. 9 or 17.

169. The non-human animal or part thereof according to any one of claims 160 to 168, wherein the non-human animal is a gene-edited non-human animal, or derived from the gene-edited non-human animal.

170. The non-human animal or part thereof according to any one of claims 160 to 169, wherein the non-human animal is a gene-knocked-in non-human animal, or is derived from a gene-knocked-in non-human animal.

171. The non-human animal or part thereof according to any one of claims 160 to 170, wherein the expression of the ATP6V1B2 and/or functional active fragments thereof is modulated by corresponding endogenous modulatory elements in the genome of the non-human animal.

172. The non-human animal or part thereof according to any one of claims 160 to 171, wherein the non-human animal is a non-human mammal.

173. The non-human animal or part thereof according to any one of claims 160 to 172, wherein the non-human animal is selected from: mouse, rat, rabbit, ape, monkey, pig, cow, sheep, horse, chicken, dog, alpaca and cat.

174. The non-human animal or part thereof according to any one of claims 160 to 173, wherein the non-human animal is a rodent or a primate.

175. The non-human animal or part thereof according to any one of claims 160 to 174, wherein the part comprises organs, tissues and/or cells of the non-human animal.

176. The non-human animal or part thereof according to any one of claims 160 to 175, wherein the part comprises body fluid.

177. The non-human animal or part thereof according to claim 176, wherein the body fluid is selected from the following groups: blood, plasma, serum, urine, sweat, tears, saliva, semen and cerebrospinal fluid.

178. The non-human animal or part thereof according to any one of claims 160 to 177, wherein the part comprises the brain or part of the brain.

179. The non-human animal or part thereof according to any one of claims 160 to 178, wherein the part is unable to develop into a complete non-human animal.

180. An isolated cell, comprising and/or expressing ATP6V1B2, a functional active fragment thereof, and/or a nucleic acid molecule encoding same.

181. The cell according to claim 180, wherein the ATP6V1B2 is derived from human.

182. The cell according to any one of claims 180 to 181, wherein the ATP6V1B2 and/or functional active fragment thereof comprise the amino acid sequence as set forth in any one of SEQ ID NO: 8, 10-11, 16.

183. The cell according to any one of claims 180 to 182, wherein the gene encoding the ATP6V1B2 and/or the functional active fragments thereof is homozygous or heterozygous.

184. The cell according to any one of claims 180 to 183, wherein the nucleic acid molecule encoding the ATP6V1B2 and/or the functional active fragments thereof comprises the nucleic acid sequence as set forth in SEQ ID NO: 9 or 17.

185. The cell according to any one of claims 180 to 184, wherein the expression of the ATP6V1B2 and/or functional active fragments thereof is modulated by corresponding endogenous modulatory elements in the genome of the cells.

186. The cell according to any one of claims 180 to 185, wherein the cell is a non-human animal cell.

187. The cell according to claim 186, wherein the non-human animal is a non-human mammal.

188. The cell according to any one of claims 180 to 187, wherein the non-human animal is selected from: mouse, rat, rabbit, ape, monkey, pig, cow, sheep, horse, chicken, dog, alpaca and cat.

189. The cell according to any one of claims 180 to 188, wherein the non-human animal is a rodent or a primate.

190. The cell according to any one of claims 180 to 189, wherein the cell is unable to develop into a complete non-human animal.

191. A tissue, comprising and/or expressing ATP6V1B2, a functional active fragment thereof, and/or a nucleic acid molecule encoding same.

192. The tissue according to claim 191, wherein the ATP6V1B2 is derived from human.

193. The tissue according to any one of claims 191 to 192, wherein the ATP6V1B2 and/or the functional active fragments thereof comprise the amino acid sequence as set forth in any one of SEQ ID NO: 8, 10-11, 16.

194. The tissue according to any one of claims 191 to 193, wherein the gene encoding the ATP6V1B2 and/or the functional active fragments thereof is homozygous or heterozygous.

195. The tissue according to any one of claims 191 to 194, wherein the nucleic acid molecule encoding the ATP6V1B2 and/or the functional active fragments thereof comprises the nucleic acid sequences as set forth in SEQ ID NO: 9 or 17.

196. The tissue according to any one of claims 191 to 195, wherein the tissue is a gene-edited tissue, or derived from gene editing.

197. The tissue according to any one of claims 191 to 196, wherein the tissue is a gene-knocked-in tissue, or is derived from a gene-knocked-in tissue.

198. The tissue according to any one of claims 191 to 197, wherein the expression of the ATP6V1B2 and/or functional active fragments thereof is modulated by corresponding endogenous modulatory elements in the genome of the tissue.

199. The tissue according to any one of claims 191 to 198, wherein the tissue is the tissue of a non-human animal.

200. The tissue according to claim 199, wherein the non-human animal is a non-human mammal.

201. The tissue according to any one of claims 191 to 200, wherein the non-human animal is selected from: mouse, rat, rabbit, ape, monkey, pig, cow, sheep, horse, chicken, dog, alpaca and cat.

202. The tissue according to any one of claims 191 to 201, wherein the non-human animal is a rodent or a primate.

203. The tissue according to any one of claims 191 to 202, wherein the tissue comprises body fluid.

204. The tissue according to claim 203, wherein the body fluid is selected from the following groups: blood, plasma, serum, urine, sweat, tears, saliva, semen and cerebrospinal fluid.

205. The tissue according to any one of claims 191 to 204, wherein the tissue comprises the brain or part of the brain.

206. The tissue according to any one of claims 191 to 205, wherein the tissue is unable to develop into a complete non-human animal.

207. A cell line or cell culture, derived from the non-human animal or part thereof according to any one of claims 160 to 179, derived from the cell according to any one of claims 180 to 190, and/or derived from the tissue according to any one of claims 191 to 206.

208. The cell line or cell culture according to claim 207, wherein the cell culture is a primary cell culture.

209. The cell line or cell culture according to any one of claims 207 to 208, wherein the cell culture comprises organoids.

210. A composition, comprising the non-human animal or part thereof according to any one of claims 160 to 179, the cell according to any one of claims 180 to 190, the tissue according to any one of claims 191 to 206, and the cell line or cell culture according to any one of claims 207 to 209.

211. A kit, comprising the non-human animal or part thereof according to any one of claims 160 to 179, the cell according to any one of claims 180 to 190, the tissue according to any one of claims 191 to 206, and/or the cell line or cell culture according to any one of claims 207 to 209, and one or more additional components selected from the following groups: analytical buffers, controls, substrates, standards, assay materials, laboratory supplies, devices, instruments, cells, organs, tissues, and user manuals or instructions.

212. A method for screening drugs capable of improving learning ability, treating cognitive impairment, and/or treating neurodegenerative diseases of a subject, comprising: applying candidate drugs to the non-human animal or part thereof according to any one of claims 160 to 179, the cell according to any one of claims 180 to 190, the tissue according to any one of claims 191 to 206, and/or the cell line or cell culture according to any one of claims 207 to 209; and detecting the influence of the candidate drugs on the expression level and/or activity of ATP6V1B2 in the subject.

213. A method for screening drugs capable of improving learning ability, treating cognitive impairment, and/or treating neurodegenerative diseases of a subject, comprising: (i) applying candidate drugs to the non-human animal or part thereof according to any one of claims 160 to 179, the cell according to any one of claims 180 to 190, the tissue according to any one of claims 191 to 206, and/or the cell line or cell culture according to any one of claims 207 to 209; (ii) detecting the influence of the candidate drugs on the expression level and/or activity of ATP6V1B2 in the subject; and (iii) in a case that the expression level and/or activity of the ATP6V1B2 is improved after applying the candidate drugs, determining that the candidate drugs is capable of improving the learning ability, treating cognitive impairment, and/or treating neurodegenerative diseases of the subject.

214. The method according to any one of claims 212 to 213, wherein the expression level of the ATP6V1B2 comprises the expression level of an ATP6V1B2 gene, the transcription level of the ATP6V1B2 gene and/or the expression level of ATP6V1B2 protein.

215. The method according to any one of claims 212 to 214, wherein the improvement comprises that the expression level and/or activity of ATP6V1B2 is improved by at least about 10% as compared with the expression level and/or activity of original ATP6V1B2 in the subject.

216. The method according to any one of claims 212 to 215, wherein the learning ability comprises cognitive ability, motor ability, memory ability, and/or spatial exploration ability.

217. The use according to any one of claims 212 to 216, wherein the cognitive impairment comprises mild cognitive impairment (MCI), middle cognitive impairment and late cognitive impairment.

218. The use according to any one of claims 212 to 217, wherein the cognitive impairment comprises cognitive impairment caused by normal aging, Lews Body Dementia (LBD), frontotemporal dementia and/or vascular dementia.

219. The use according to any one of claims 212 to 218, wherein the diseases induced by the cognitive impairment comprises Alzheimer disease, multi-infarct, Parkinson's disease, AIDS and/or Creutzfeldt-Jakob disease (CJD).

220. The use according to any one of claims 212 to 219, wherein the neurodegenerative disease comprises acute neurodegenerative disease and chronic neurodegenerative disease.

221. The use according to any one of claims 212 to 220, wherein the neurodegenerative disease comprises neurodegenerative disease caused by neuronal death and glial cell homeostasis, neurodegenerative disease caused by aging, neurodegenerative disease caused by influence on the CNS cell function, neurodegenerative disease caused by abnormal communication between cells, and/or neurodegenerative disease caused by damage to cell motor.

222. The use according to any one of claims 212 to 221, wherein the neurodegenerative disease comprises Alzheimer disease, Parkinson's disease, multiple sclerosis (MS), amyotrophic lateral sclerosis (ALS), and/or Huntington's disease (HD).

223. A use of the non-human animal or part thereof according to any one of claims 160 to 179, the cell according to any one of claims 180 to 190, the tissue according to any one of claims 191 to 206, and/or the cell line or cell culture according to any one of claims 207 to 209 in preparing an identification and/or screening system, wherein the system is used for screening drugs and/or biomarkers capable of improving learning ability, treating cognitive impairment, and/or treating neurodegenerative diseases of a subject.

224. The non-human animal or part thereof according to any one of claims 160 to 179, the cell according to any one of claims 180 to 190, the tissue according to any one of claims 191 to 206, and/or the cell line or cell culture according to any one of claims 207 to 209, being used for screening of drugs and/or biomarkers capable of improving learning ability, treating cognitive impairment, and/or treating neurodegenerative diseases of a subject.

225. A method for preparing the non-human animal or part thereof according to any one of claims 160 to 179, the cell according to any one of claims 180 to 190, the tissue according to any one of claims 191 to 206, comprising: enabling the non-human animal or part thereof, cell or tissue to comprise and/or express ATP6V1B2 and/or functional active fragments thereof.

226. A method for preparing a disease model, comprising: enabling a non-human animal or part thereof, cell or tissue to comprise and/or express ATP6V1B2 and/or functional active fragments thereof.

227. The method according to any one of claims 225 to 226, wherein the ATP6V1B2 and/or the functional active fragments thereof comprise the amino acid sequence as set forth in any one of SEQ ID NO: 8, 10-11, 16.

228. The method according to any one of claims 225 to 227, comprising: transfecting the nucleic acid sequence encoding the ATP6V1B2 and/or functional active fragments thereof.

229. The method according to claim 228, wherein the nucleic acid sequence encoding the ATP6V1B2 and/or functional active fragments thereof comprise the nucleic acid sequence as set forth in SEQ ID NO: 9 or 17.

230. The method according to any one of claims 228 to 229, wherein the transfecting comprises: transfecting adeno-associated virus comprising the nucleic acid sequence.
